# EUROPÄISCHE PATENTANMELDUNG

(11) **EP 4 555 891 A2**
(43) Veröffentlichungstag der Anmeldung: **21.05.2025**
(21) Anmeldenummer: 25167801.7
(22) Anmeldetag: 17.02.2023
(51) Int. Cl.: A45C 13/02

(54) **ERSTE-HILFE-KOFFER, NOTFALLKOFFER, ERSTE-HILFE-KOFFERHÄLFTE, NOTFALLKOFFERHÄLFTE, SYSTEM VON NOTFALLKOFFERN UND/ODER NOTFALLKOFFERHÄLFTEN SOWIE TRENNELEMENT**

(30) Priorität: 23.12.2022 EP 22216544; 23.01.2023 EP 23152967; 06.02.2023 EP 23155219
(62) Teilanmeldung aus: 23157243.9
(71) Anmelder: W. Söhngen GmbH, 65232 Taunusstein (DE)
(72) Erfinder: Arnold, Pascal, 66265 Heusweiler (DE); May, Melanie, 65193 Wiesbaden (DE)
(74) Vertreter: Hoffmann, Jürgen

(57) **Zusammenfassung**

Die Erfindung betrifft ein System von Notfallkoffern in verschiedenen Größen und/oder von Notfallkofferhälften in verschiedenen Größen, wobei die Notfallkoffer jeweils zwei Notfallkofferhälften haben, die je eine Kofferschale aufweisen, wobei in zumindest einer Kofferschale Trennelemente zur Bildung von Fächern bereitgestellt sind, wobei unmittelbar als Teil von zumindest zwei Innenwänden dieser Kofferschale je eine Folge von Steckeinrichtungen bereitgestellt ist und wobei die Trennelemente zumindest in einem Teil der Steckeinrichtungen eingesteckt sind, bzw. wobei die Notfallkofferhälften jeweils eine Kofferschale und eine unmittelbar als Teil von zumindest zwei Innenwänden der Kofferschale jeweils angeordneten Folge von Steckeinrichtungen haben, wobei in zumindest einem Teil der Steckeinrichtungen Trennelemente zur Bildung von Fächern eingesteckt sind. Die Abmessungen der Kofferschalen sind mit den Steckeinrichtungen untereinander und mit den Abmessungen der Trennelemente derart aufeinander abgestimmt, dass vorbestimmte Trennelemente in mehreren Notfallkoffern und/ oder Notfallkofferhälften mit unterschiedlich großen Kofferschalen nutzbar sind.

## Beschreibung

Die Erfindung betrifft einen Erste-Hilfe-Koffer, Notfallkoffer, und sie betrifft eine zum Zusammenbau dieses verwendbare Erste-Hilfe-Koffer-Hälfte, Notfallkofferhälfte. Sie betrifft zudem ein System von Notfallkoffern in verschiedenen Größen und/oder von Notfallkofferhälften in verschiedenen Größen sowie ferner ein für die Erste-Hilfe-Koffer, Notfallkoffer, bzw. Erste-Hilfe-Kofferhälften, Notfallkofferhälften, verwendbares Trennelement.

Unter Notfallkoffer wird hier vorliegend insbesondere verstanden ein Koffer, der befüllt ist mit zumindest einem aus: Pflaster und Verbandsmaterial, Einmalhandschuhen, Erste-Hilfe-Scheren und Pinzetten, Hygienemundschutz, Hygienekleidung, Notduschen und Augenspülung, Desinfektionsmittel und Spender dazu, Defibrillatoren und Zubehör, sowie Beatmungsmasken. Ein Erste-Hilfe-Koffer ist vorliegend als unter den Begriff des Notfallkoffers im Sinne der Erfindung fallend anzusehen.

Die Erfindung betrifft insbesondere das Vorsehen von Fächern in zumindest einer Kofferschale des Notfallkoffers.

Aus der EP 0 691114 A2 ist ein Notfallkoffer bekannt, der zwei klappbar miteinander verbundene Hartschalen aufweist. In die Hartschalen werden Einsätze eingesetzt, wobei wiederum in die Einsätze Trennwende einsetzbar sind, um die Einsätze in Fächer zu unterteilen.

Die DE 88 14 319 U1 beschreibt das Vorsehen von Trennwänden in einem Behälter zur Aufnahme von Erste-Hilfe-Materialien, wobei zur Befestigung der Trennwände Rasterleisten an der Innenseite der Seitenwände angelegt werden.

Es ist Aufgabe der Erfindung, einen verbesserten Notfallkoffer zu schaffen und entsprechende Nutzungen zu ermöglichen.

Die Aufgabe wird durch einen Notfallkoffer mit den Merkmalen nach Anspruch 1, durch eine Notfallkofferhälfte mit den Merkmalen nach Anspruch 10, durch ein System nach Anspruch 13 sowie ein Trennelement nach Anspruch 15 gelöst.

Der erfindungsgemäße Notfallkoffer umfasst somit zwei Notfallkofferhälften, die je eine Kofferschale aufweisen. In zumindest einer Kofferschale sind Trennelemente zur Bildung von Fächern bereitgestellt, wobei unmittelbar als Teil von zumindest zwei Innenwänden dieser Kofferschale (je) eine Folge von Steckeinrichtungen bereitgestellt ist, und wobei die Trennelemente zumindest in einem Teil der Steckeinrichtungen eingesteckt sind.

Durch die Bereitstellung der Steckeinrichtungen unmittelbar als Teil von den Innenwänden (d.h. unmittelbar an den Innenwänden), also nicht mehr unter Bereitstellung der Steckeinrichtungen an anderen in die Kofferschale eingesetzten Elementen wie im Falle der Rasterleiste aus der DE 88 14 319 U1, oder der Einsätze aus der EP 0 691 114 A2, können die Steckeinrichtungen unmittelbar mit Herstellung der Kofferschalen vorgesehen werden. Dadurch können Herstellungsschritte (Anlegen und Befestigen der Rasterleisten/Einsetzen der Einsätze) vermieden werden. Auch kann die Anordnung mit weniger Aufwand stabil hergestellt sein als im Stand der Technik.

Gemäß Ausführungsformen der Erfindung weist der Notfallkoffer rechteckige Kofferschalen auf. Auf diese Weise können geradlinig verlaufende Trennelemente je an einander gegenüberliegenden Steckeinrichtungen einer Kofferschale eingesteckt sein.

Rechteckige Kofferschalen sind weit verbreitet und lassen sich leicht unterbringen, z.B. leichter stapeln als andere Formen. Dass die Trennelemente geradlinig sind, hat den Vorteil einer erleichterten Handhabbarkeit.

Gemäß vorteilhaften Ausführungsformen entspricht zumindest einer Steckeinrichtung als Teil der Innenwand der Kofferschale eine Verstärkungsrippe an der Außenwand örtlich, und vorzugsweise entspricht jeder Steckeinrichtung an der Innenwand der Kofferschale eine Verstärkungsrippe an der Außenwand örtlich. Somit lässt sich die Steckeinrichtung bereitstellen, ohne dass die Kofferschale an Stabilität verliert.

Bei diesen und/oder anderen Ausführungsformen ist es vorteilhaft, wenn jede Kofferschale in einem Stück aus Hartplastik hergestellt ist, z.B. durch Spritzguss. Gleiches gilt für die Trennelemente, die jeweils einzeln in einem Stück aus Hartplastik hergestellt sein können. Dadurch sind die Trennelemente besonders stabil. Das Material für die Trennelemente kann von dem für die Kofferschale unterschiedlich sein, etwa können die Trennelemente transparent oder teiltransparent sein, damit man einen besseren Überblick über den Inhalt der einzelnen Fächer erhält.

Die Trennelemente weisen gemäß vorteilhaften Ausführungsformen einen streifenförmigen Grundkörper auf, wobei zumindest an einer Seite des streifenförmigen Grundkörpers eine Folge von Steckeinrichtungen für andere Trennelemente vorgesehen ist. Dadurch können kleinteilig Fächer bereitgestellt werden.

Vorteilhaft ist es in diesem Zusammenhang, wenn die Steckeinrichtungen der Trennelemente jeweils mehrere Nasen aufweisen, welche alternierend rechts und links untereinander angeordnet sind. Bereits diese einfache Ausführungsform ist unaufwändig herstellbar und kann stabil gestaltet sein.

Gemäß vorteilhaften Ausführungsformen weisen die Trennelemente an zumindest einem Längsende eine Schiebeschiene auf, die in eine Steckeinrichtung einbringbar ist. Besonders vorteilhaft ist es, wenn die Schiebeschiene sowohl in Steckeinrichtungen an den Innenwänden der Kofferschalen als auch an Steckeinrichtungen anderer Trennelemente einbringbar sind, die Trennelemente universell einsetzbar sind.

Bei dem erfindungsgemäßen Notfallkoffer sind gemäß Ausführungsformen der Erfindung in jeder Kofferschale Trennelemente zur Bildung von Fächern bereitgestellt, und die obigen Angaben zu den Kofferschalen, ihrer Herstellung aus Hartplastik und zu den Trennelementen gelten gleichermaßen für beide Kofferschalen bzw. die darin befindlichen Trennelemente.

Die erfindungsgemäße Notfallkofferhälfte umfasst eine Kofferschale und eine unmittelbar als Teil von zumindest zwei Innenwänden der Kofferschale angeordnete Folge von (jeweiligen) Steckeinrichtungen, wobei in zumindest einem Teil der Steckeinrichtungen Trennelemente zur Bildung von Fächern eingesteckt sind. Die zum Notfallkoffer beschriebenen Vorteile und vorteilhaften Ausführungsformen gelten gleichermaßen zur Notfallkofferhälfte.

Vorteilhafterweise ist in diesem Zusammenhang vorgesehen, dass in einem Teil der Steckeinrichtungen Trennelemente zur Bildung von Fächern eingesteckt sind, nicht aber in einem anderen Teil. Auf diese Weise lassen sich die Trennelemente gegebenenfalls umstecken. Die Folge von Steckeinrichtungen je an einer Innenwand, vorzugsweise je Innenwand eine Folge von Steckeinrichtungen, ermöglicht eine flexible Nutzung.

Die Notfallkofferhälfte weist gemäß Ausführungsformen einen Innendeckel auf, wobei der Innendeckel zur Herstellung einer geschlossenen Notfallkofferhälfte an der Kofferschale arretierbar ist.

Durch den Innendeckel lässt sich die Notfallkofferhälfte auch einzeln transportieren.

Wegen des arretierbaren Innendeckels fällt das in der jeweiligen Notfallkofferhälfte befindliche Material dabei auch nicht heraus.

Im Zusammenhang mit dem Notfallkoffer ist besonders vorzugsweise vorgesehen, dass der Notfallkoffer zwei Notfallkofferhälften aufweist, die je eine Kofferschale und einen Innendeckel aufweisen, wobei die Notfallkofferhälften lösbar verbunden sind und wobei jeder Innendeckel zur Herstellung einer geschlossenen Notfallkofferhälfte an seiner Kofferschale arretierbar ist. Diese Ausführungsform kann von Vorteil sein unter anderem dann, wenn nur eine Kofferschale benötigt wird, oder wenn zwei Ersthelfer bei einem Patienten gemeinsam über einen Notfallkoffer der genannten Art verfügen und sich jeweils aus einer der Kofferschalen mit dem darin befindlichen Material bedienen möchten.

Der Notfallkoffer ist somit flexibel einsetzbar: Die beiden Notfallkofferhälften können verbunden bleiben; dann hat der behandelnde Ersthelfer, Arzt oder sonstiges Bedienpersonal einen großen Notfallkoffer mit viel Material. Bei gleicher Bestückung der Notfallkofferhälften sind dann etwa besonders viele Verbandsmaterialien vorhanden. Bei unterschiedlicher Bestückung der Notfallkofferhälften können sich die Inhalte einander ergänzen. Wenn die Notfallkofferhälften getrennt werden, können sie jeweils von zwei verschiedenen Bedienpersonen verwendet werden. Das Auftrennen kann gegebenenfalls schnell vor Ort erfolgen. Bei Vorhandensein eines Notfallkoffers können somit zwei Bedienpersonen jeweils eine Notfallkofferhälfte verwenden und entsprechend zwei Patienten gleichzeitig behandeln.

Gemäß einer bevorzugten Ausführungsform sind die Notfallkofferhälften ausschließlich durch an den Kofferschalen befindliche Mittel lösbar verbunden. Hier können insbesondere Hilfsmittel wie Schrauben und dergleichen entfallen. Typischerweise ist bei dieser Ausführungsform dafür gesorgt, dass die Notfallkofferhälften besonders schnell voneinander lösbar sind, so dass vor Ort die oben beschriebene Auftrennung des Notfallkoffers für zwei Bedienpersonen kurzfristig möglich sein kann.

Gemäß einer weiteren bevorzugten Ausführungsform weist der Notfallkoffer zwei gleich gebaute Kofferschalen auf. Dies kann nicht nur den Vorteil der Symmetrie bergen, sondern auch die gegebenenfalls an der Kofferschale zum Verbinden befindlichen Mittel können zueinander komplementär gestaltet werden. Zur Herstellung eines Notfallkoffers müssen dann nicht zwei unterschiedliche Kofferschalen bereitgestellt werden.

Vorzugsweise ist in diesem Zusammenhang vorgesehen, dass an jeder Kofferschale eine Stange und dazu achsgleich auf Umschlag eine Steckvorrichtung für die Stange der anderen Kofferschale angeordnet sind. Man muss dann nur die Stange in die, im Querschnitt vorzugsweise C-förmige, Steckvorrichtung einbringen (hineindrücken oder -stecken), um die Notfallkofferhälften miteinander zu verbinden bzw. umgekehrt lässt sich bei geeigneter Elastizität der Steckvorrichtung die Stange auch wieder lösen. Tut man dies bei beiden Steckvorrichtungen für die jeweiligen Stangen, kann man den Notfallkoffer in die beiden Notfallkofferhälften auftrennen.

Weiter bevorzugt ist hierzu vorgesehen, dass zwischen der Stange und der Steckvorrichtung achsgleich verlaufende Scharniere für den jeweiligen Innendeckel der Kofferschale angeordnet sind, wobei die Scharniere der einen Kofferschale in Lücken zwischen den Scharnieren der anderen Kofferschale greifen. Dies kann insbesondere so ausgestaltet sein, dass die Lücken bis auf ein geringes Spiel vollständig geschlossen werden (ggf. kann sogar ein Einrasten vorgesehen sein); so wird eine nahezu durchgängige Achse für die Kofferschalen einerseits und für den Innendeckel andererseits bereitgestellt, über die eine Drehung der Kofferschalenhälften zueinander bzw. des Innendeckels einzeln ermöglicht ist.

Das erfindungsgemäße System von Notfallkoffern mit den Merkmalen nach den beschriebenen Ausführungsformen und/oder von Notfallkofferhälften mit den Merkmalen nach den beschriebenen Ausführungsformen, wobei die Notfallkoffer bzw. Notfallkofferhälften in verschiedenen Größen vorliegen, hat die Eigenschaft, dass die Abmessungen der Kofferschalen mit den Steckeinrichtungen untereinander und mit den Abmessungen der Trennelemente derart aufeinander abgestimmt sind, dass vorbestimmte Trennelemente in mehreren Notfallkoffern und/oder Notfallkofferhälften mit unterschiedlich großen Kofferschalen nutzbar sind.

Wer das erfindungsgemäße System der herstellt, muss also die Trennelemente nur einmal in einer, zwei, drei, vier, fünf oder gegebenenfalls auch mehr Größen vorsehen, kann aber alle diese Trennelemente bevorzugt in mehreren Notfallkoffern/Notfallkofferhälften (oder in einem Notfallkoffer und einer Notfallkofferhälfte mit unterschiedlichen Größen) einsetzen. Durch diese Modularität ist die Herstellung von Notfallkoffern bzw. Notfallkofferhälften erleichtert.

Dies kann auf vorteilhafte Weise möglich sein, wenn die Trennelemente durchgängig eine gleiche Höhe aufweisen und wenn auch die Kofferschalen bei unterschiedlichen Längen-Breite-Verhältnissen alle die gleiche Höhe aufweisen oder zumindest annäherungsweise gleich hoch sind, damit Trennelemente gleicher Höhe bei allen verschiedenen Notfallkoffern bzw. Notfallkofferhälften einsetzbar sind.

Gemäß vorteilhaften Ausführungsformen zum erfindungsgemäßen System ist vorgesehen, dass die vorbestimmten Trennelemente eine Gruppe als Bausatz bilden, der bei allen Notfallkoffern oder Notfallkofferhälften des Systems nutzbar ist. Dies ist, wie eben ausgeführt, in vorteilhafter Weise mit der Nutzung von Trennelementen gleicher Höhe verbunden.

Das erfindungsgemäße Trennelement für eine Kofferschale eines Notfallkoffers umfasst einen streifenförmigen Grundkörper, an welchem Steckeinrichtungen ausgebildet sind, wobei jede Steckeinrichtung Nasen aufweist, welche alternierend rechts und links voneinander angeordnet sind.

Die Vorteile dieses Trennelements wurden bereits oben unter Bezug auf den Notfallkoffer beschrieben.

Gemäß vorteilhaften Ausführungsformen hierzu sind auf jeder Seite des streifenförmigen Grundkörpers Steckeinrichtungen bereitgestellt. Jeder Nase der einen Seite liegt eine Aussparung auf dieser einen Seite gegenüber, wobei dieser gegenüberliegenden Aussparung eine Nase an der anderen Seite entspricht. Der Nase an der einen Seite wiederum entspricht die entsprechende Aussparung auf der anderen Seite. Die entsprechende Aussparung auf der anderen Seite liegt entsprechend also auch der Nase auf der anderen Seite gegenüber. Bei dieser Ausführungsform werden somit einfach "hohle Nasen" bereitgestellt, wobei das Innere der Nase der Aussparung auf der anderen Seite entspricht. Von oben gesehen bilden die Nasen auf der einen Seite der Trennelemente und die Nasen auf deren anderen Seite eine Art Zickzack-Muster. Dadurch, dass die Nasen alternierend rechts und links untereinander angeordnet sind, sind auch die Nasen auf der anderen Seite rechts und links alternierend untereinander angeordnet, denn jeder Aussparung links entspricht die Nase rechts auf der anderen Seite. Diese Ausführungsform kann, wie oben bereits mitgeteilt, durch Spritzguss aus Hartplastik, insbesondere transparentem oder semitransparentem Kunststoff, bereitgestellt sein.

Gemäß Ausführungsformen der Erfindung in einem Aspekt umfasst der Notfallkoffer eine erste Kofferschale und eine zweite Kofferschale und eine erste Handhabeeinrichtung an der ersten Kofferschale, welche aus einer Ruhestellung in eine Schließstellung verbringbar ist und umgekehrt, und umfasst ferner eine zweite Handhabeeinrichtung an der zweiten Kofferschale, welche ebenfalls aus einer Ruhestellung in eine Schließstellung verbring-bar ist und umgekehrt, wobei bei gegebener Ruhestellung beider Handhabeeinrichtungen der Notfallkoffer geöffnet ist und wobei in der Schließstellung der ersten Handhabeeinrichtung die erste Handhabeeinrichtung mit einem Hintergreifelement einen an der zweiten Kofferschale ausgebildeten Körper hintergreift und wobei in der Schließstellung der zweiten Handhabeeinrichtung die zweite Handhabeeinrichtung mit einem Hintergreifelement einen an der ersten Kofferschale ausgebildeten Körper hintergreift.

Bei dieser Ausführungsform wird somit davon abgegangen, die Handhabeeinrichtungen beide an einer Kofferschale vorzusehen. Somit ist die Stabilität der Anordnung erhöht. Der Notfallkoffer ist somit besonders solide gebaut.

Gemäß einer bevorzugten Ausführungsform zu diesem Aspekt ist jede Handhabeeinrichtung drehbar. Das Hintergreifen eines Körpers mit einem Hintergreifelement ist besonders einfach umsetzbar, wenn das Hintergreifelement hinter den Körper gedreht wird, also die gesamte Handhabeeinrichtung drehbar ist.

Bei einer bevorzugten Ausführungsform hierzu ist des Weiteren vorgesehen, dass jede Handhabeeinrichtung bei nach oben offener zugehöriger Kofferschale vorne links angeordnet ist (bei nach unten offener zugehöriger Kofferschale also vorne rechts) und sich gegen den Uhrzeigersinn von der Ruhestellung in die Schließstellung drehen lässt. Wenn die Handhabeeinrichtung links angeordnet ist und sich dann gegen den Uhrzeigersinn drehen lässt, entspricht dies einer Bewegung nach außen hin, die sich aus dem Handgelenk bzw. Ellenbogen heraus leicht vornehmen lässt. Die zugleich vorzunehmende Schließung der zweiten Handhabeeinrichtung von vorne rechts gegen den Uhrzeigersinn nach unten kann praktisch synchron aus dem rechten Handgelenk heraus nach unten erfolgen. Dieser Schließvorgang ist besonders intuitiv. Der umgekehrte Öffnungsvorgang (rechte Hand dreht sich nach oben, linke nach unten) ist zudem hilfreich.

Gemäß einer weiteren vorteilhaften Ausführungsform zum Aspekt mit der Handhabeeinrichtung kann jede Handhabeeinrichtung eine Handhabe umfassen, zu der ein geometrischer Mittelpunkt definierbar ist. Eine Drehachse der Handhabeeinrichtung liegt nun zum geometrischen Mittelpunkt der Handhabe exzentrisch.

In diesem Aspekt ist die Handhabe wesentlich besser schwenkbar und dadurch leichter handhabbar. Dieser Aspekt kann unabhängig von dem anderen Aspekt mit der Handhabeeinrichtung seinerseits einen eigenen Erfindungsaspekt darstellen: Notfallkoffer mit einer ersten Kofferschale und einer zweiten Kofferschale und mit einer Handhabeeinrichtung an jeder Kofferschale, wobei jede Handhabeeinrichtung eine Handhabe umfasst, zu der ein geometrischer Mittelpunkt definierbar ist, und wobei eine Drehachse der Handhabeeinrichtung zum geometrischen Mittelpunkt der Handhabe exzentrisch liegt.

Gemäß bevorzugten Ausführungsformen zu allen genannten Aspekten der Erfindung wie oben beschrieben sowie zu weiteren Aspekten der Erfindung wie weiter unten genannt, weist jede Handhabeeinrichtung eine kreuzförmige Handhabe auf. Im Unterschied zu Rädern ist die Kreuzform hier vorteilhaft, weil anhand der Kreuzform - insbesondere in Verbindung auch mit der exzentrischen Lagerung der Handhabeeinrichtung - besser ersichtlich ist, in welcher Position sich die Handhabeeinrichtung befindet.

Vorzugsweise ist hierzu vorgesehen, dass gleich lange Schenkel der Kreuzform in einem Winkel von 90° zum jeweils benachbarten Schenkel stehen. (Das Kreuz weist also genau vier gleiche Schenkel auf.) Dies erleichtert die Bedienung.

Nach Ausführungsformen hierzu kann eine (Dreh-)Welle der Handhabeeinrichtung an einem ersten Schenkel der Kreuzform ausgebildet sein, wodurch die oben genannte Ausführungsform der exzentrischen Lagerung verwirklicht sein kann.

Das Kreuz wird also nicht um den Mittelpunkt gedreht, von dem die vier Schenkel abstehen, also nicht um den geometrischen Mittelpunkt, sondern um einen der Schenkel, so dass der Schwenkbereich der Handhabeeinrichtung vergrößert ist, was seinerseits die Bedienung erleichtert. Dies gilt insbesondere in Verbindung mit der Ausführungsform der Drehbarkeit zum Schließen im Uhrzeigersinn bei links vorne angeordneter Handhabeeinrichtung (bezüglich der offenen Kofferschale).

Vorteilhafterweise kann hierzu vorgesehen sein, dass das Hintergreifelement an dem dem ersten Schenkel gegenüberliegenden Schenkel der Kreuzform ausgebildet ist. Hier wird auch der Vorteil der Kreuzform ersichtlich: Die Kreuzform kann aufgrund von Seitenwänden an den Schenkeln besonders stabil ausgebildet sein, was ebenfalls die Handhabbarkeit erleichtert.

Gemäß Ausführungsformen in allen beschriebenen Aspekten mit Handhabeeinrichtung kann der von einer Handhabeeinrichtung hintergriffene oder hintergreifbare Körper als Nase ausgebildet sein. Vorzugsweise weist er hierzu einen Rastmechanismus auf. Die Nase hat, anders als ein einfacher Zapfen, den Vorteil einer Stabilität des Verschlusses, da die beiden Kofferschalen so auf gewisse Weise ineinander verhakt werden. Durch den Rastmechanismus wird der Halt gegebenenfalls noch verstärkt.

Gemäß vorteilhaften Ausführungsformen zu dem Aspekt mit der Handhabeeinrichtung gibt es neben der Ruhestellung und der Schließstellung noch eine Parkstellung. Dies lässt sich so beschreiben, dass jede Handhabeeinrichtung zur Herstellung der Schließstellung ausgehend von der Ruhestellung in eine erste Drehrichtung, vorzugsweise um 90°, zu verdrehen ist, und wobei jede Handhabeeinrichtung ausgehend von der Ruhestellung in eine zweite Drehrichtung entgegengesetzt zur ersten Drehrichtung zur Herstellung einer Parkstellung verdrehbar ist, vorzugsweise um 90°.

Dies kann den Vorteil haben, dass das Hintergreifelement weniger stört, um das Herausnehmen von Gegenständen aus dem Notfallkoffer zu erleichtern. Diese Ausführungsform ist in besonderem Maße vorteilhaft in Verbindung mit der exzentrischen Lagerung der Handhabeeinrichtung, und weiter insbesondere dies auch in Verbindung mit der Kreuzform. Wenn das Kreuz verschwenkt wird, kann es in der Parkstellung seinerseits besonders wenig stören.

Gemäß einer weiteren bevorzugten Ausführungsform weist zumindest eine der Notfallkofferhälften einen Griff an der Kofferschale auf. Mittels eines Griffs lässt sich der Notfallkoffer besonders gut tragen.

Hierzu weisen beide Notfallkofferhälften einen Griff auf; dann lassen sich die beiden Notfallkofferhälften jeweils einzeln nach der Lösung voneinander tragen.

Alternativ weist nur eine Notfallkofferhälfte einen Griff auf. Dies kann dann sinnvoll sein, wenn in der jeweiligen Kofferschale besonders wichtiges Material gelagert ist und wenn in der anderen Kofferschale nicht immer zu verwendendes Material gelagert ist. Dann lässt sich die Notfallkofferhälfte mit dem wichtigeren Material abtrennen und die andere Notfallkofferhälfte gegebenenfalls nur bei Bedarf heranziehen. Vorzugsweise ist hierbei an der Kofferschale ohne Griff vorgesehen, dass an der Stelle, wo sonst der Griff ein- oder angreift, Blindkappen vorgesehen sind.

Vorzugsweise ist jeder Griff mit zwei Ansatzenden jeweils an einer oberen Außenecke der Kofferschale angeordnet. Der Begriff der "oberen Außenecke" bezieht sich auf eine stehende Position des Notfallkoffers. Ausgehend von jeder der oberen Außenecken erstreckt sich der Griff in einem ersten Abschnitt vertikal nach oben. (Da es zwei Außenecken gibt, gibt es zwei erste Abschnitte). In einem auf den ersten Abschnitt vorzugsweise folgenden zweiten Abschnitt erstreckt sich der Griff zu der den (Außen-) Ecken abgewandten Innenlängskante der Kofferschale hin. (Da sich der erste Abschnitt vertikal nach oben erstreckt, ist hierunter zu verstehen, dass sich der zweite Abschnitt ebenfalls oberhalb der Kofferschalenoberseite erstreckt, wobei sich das Erstrecken zur Innenlängskante der Kofferschale hin auch so formulieren lässt, dass in Draufsicht der zweite Abschnitt die Innenlängskante erreicht). Ein die beiden zweiten Abschnitte verbindender dritter Abschnitt des Griffs erstreckt sich des Weiteren entlang der Innenlängskante. Mit anderen Worten erstreckt sich der Griff ausgehend von einer ersten oberen Außenecke in einem ersten Abschnitt vertikal nach oben, dann zur Innenlängskante im zweiten Abschnitt, dann entlang der Innenlängskante im dritten Abschnitt, dann wieder zurück im zweiten Abschnitt zur Außenseite der Kofferschale hin, und dann vertikal nach unten zur zweiten Außenecke hin.

Ein solcher Notfallkoffer hat den Vorteil, dass durch die Befestigung des Griffs an den Außenecken einerseits und des Vorhandenseins des dritten Abschnitts entlang der Innenlängskante zum einen der Transport erleichtert ist, weil der Griff selbst für ein Austarieren des am dritten Abschnitt mit der Hand gehaltenen Koffers sorgt. Zudem ist die Verwendung ermöglicht, dass ausgehend von der Situation, dass der Notfallkoffer auf einer Außenseite der Kofferschale liegt bzw. mit einer Außenseite der Kofferschale auf dem Boden oder einer sonstigen Unterlage aufliegt, kann die Bedienperson den Koffer mit der Hand an dem dritten Abschnitt ergreifen und zu sich hin ziehen. Diese Verwendung ist bei herkömmlichen Notfallkoffern mit etwa abgerundeten Griffen keineswegs so möglich.

Notfallkoffer müssen sich leicht tragen lassen und schnell zur Hand sein. Dies gilt auch für diesen Notfallkoffer gemäß der bevorzugten Ausführungsform: Er kann auch dann, wenn ein behandelnder Ersthelfer vor einem Patienten steht oder kniet, umstandslos beigezogen werden.

Der Arzt oder sonstiges Krankenwagenpersonal als Ersthelfer oder dergleichen kann den Koffer insbesondere neben einem Patienten auf dem Boden abstellen, sich dann zum Patienten hinknien und durch Ausstrecken des Arms den Koffer zu sich heranziehen. Dadurch kann wertvolle Zeit bei der Behandlung des Patienten gewonnen werden.

Gemäß einer bevorzugten Ausführungsform in diesem Aspekt ist der Übergang zwischen dem ersten Abschnitt und dem zweiten Abschnitt abgerundet, damit es keine störenden Ecken gibt. Alternativ oder zusätzlich ist der Übergang zwischen dem zweiten Abschnitt und dem dritten Abschnitt abgerundet, damit es keine störenden Ecken gibt.

Der zweite Abschnitt muss nicht unmittelbar von der Stelle, an der der erste Abschnitt endet, zur Innenlängskante hin verlaufen. Vielmehr kann es vorteilhaft sein, wenn der zweite Abschnitt des Griffs mit einem Teilabschnitt (also partiell) zunächst noch parallel zu einer Außenlängskante der Kofferschale verläuft und sich erst in einem weiteren Teilabschnitt geradlinig zu der Innenlängskante hin erstreckt, wobei der Teilabschnitt mit geradliniger Erstreckung nicht notwendigerweise senkrecht zu der Außenlängskante stehen muss, sondern in einem geeigneten Winkel von beispielsweise 15 bis 30° stehen kann. Dadurch gewinnt der Koffer nicht nur ein gefälliges Aussehen, sondern funktionell ist das Ergreifen von oben erleichtert, und zugleich ist auch ein Gleichgewichtsabgleich beim Halten des Koffers gewährleistet.

Der dritte Abschnitt kann sich mittig entlang der Innenlängskante erstrecken. Naturgemäß ist dies eine besonders stabile Ausführungsform, wenn der dritte Abschnitt, der gerne von der Bedienperson ergriffen wird, mittig verläuft.

Weiter bevorzugt ist dann jeder Griff auch symmetrisch ausgebildet, d.h. die ersten Abschnitte und die zweiten Abschnitte sind zueinander spiegelbildlich, der dritte Abschnitt verbindet diese. Die Spiegelebene schneidet den dritten Abschnitt in zwei Hälften.

Wenn an beiden Kofferschalen je ein Griff vorgesehen ist, wobei vorzugsweise die Griffe zueinander symmetrisch sind, also spiegelgleich ausgestaltet sind, lässt sich der gesamte Notfallkoffer besonders stabil tragen. Dies gilt in besonderem Maße, wenn die dritten Abschnitte der Griffe einander berühren.

Vorzugsweise ist weiterhin vorgesehen, dass am Ende jedes Griffs eine Stapelzentriereinrichtung ausgebildet ist.

Gemäß einer weiteren bevorzugten Ausführungsform der Erfindung sind Stapelzentriereinrichtungen an der Kofferschale und/oder am Innendeckel ausgebildet, wobei diese vorzugsweise auf Umschlag angeordnet sind.

Unter "Stapelzentriereinrichtung" wird vorliegend verstanden, dass mehrere gleichartige Notfallkoffer oder Kofferschalen zueinander komplementäre Stapelzentriereinrichtungen aufweisen können, so dass durch Formschluss, wenn nicht gar sogar Rastschluss, eine stabile Stapelung ermöglicht ist. Vorzugsweise ist hierbei vorgesehen, dass am Ende jedes Griffs eine Stapelzentriereinrichtung ausgebildet ist.

Die Stapelzentriereinrichtungen am Ende jedes Griffs, also an den Außenecken, entsprechen naturgemäß den Stapelzentriereinrichtungen eines zweiten Notfallkoffers, dort auch an den Außenecken. Auch an den unteren Außenecken der Kofferschale können Stapelzentriereinrichtungen vorgesehen sein. Dann kann es unerheblich sein, wie herum die Notfallkoffer ausgerichtet sind, die gestapelt werden.

Auch an den Blindkappen kann jeweils eine Stapelzentriereinrichtung ausgebildet sein, die dann eben mit den Stapelzentriereinrichtungen anderer Blindkappen oder an den Enden der Griffe zueinander komplementär ist.

Vorzugsweise sind die Stapelzentriereinrichtungen einer Kofferschale an den oberen Außenecken zueinander komplementär (auf Umschlag). Dann lassen sich gleichartige Kofferschalen gut aufeinanderstapeln.

Auch an jedem Innendeckel können Stapelzentriereinrichtungen vorgesehen sein, so dass die Kofferschalen einzeln stapelbar sind.

Gemäß einer bevorzugten Ausführungsform der Erfindung ist der Innendeckel an der zugehörigen Kofferschale verrastet oder verrastbar. Dies sorgt für erhöhten Schutz des in der Kofferschale befindlichen Materials: Bei verrastetem Innendeckel ist ein Herausfallen besonders unwahrscheinlich.

Ferner ist gemäß einer bevorzugten Ausführungsform vorgesehen, dass jeder Innendeckel (bei geeignet zumindest partiell geöffnetem Koffer) um 90° hochstellbar ist. Somit lässt sich jede Notfallkofferhälfte in gewisser Weise aufklappen, und der behandelnde Arzt oder die sonstige Bedienperson als Ersthelfer kann das darin befindliche Material leicht herausnehmen.

Alternativ oder zusätzlich ist es möglich, dass bei geöffnetem Koffer ein Innendeckel (und vorzugsweise ein beliebiger der beiden Innendeckel) um 180° drehbar ist, so dass er auf dem anderen Innendeckel liegt. Auf diese Weise stört der Innendeckel nicht und es kann Material aus der einen Notfallkofferhälfte leicht entnommen werden.

Wahlweise sind die beiden Merkmale des Hochstellens um 90° und des Verdrehens um 180° beide verwirklichbar, so dass wahlweise aus beiden Notfallkofferhälften Material entnommen werden kann oder der Innendeckel nicht stört und nur aus einer Notfallkofferhälfte Material entnommen wird.

Vorzugsweise ist bei diesen beiden Ausführungsformen des Stellens um 90° und des Drehens um 180° vorgesehen, dass die Innendeckel miteinander verrasten. Die Verrastbarkeit kann insbesondere unter Nutzung der vorzugsweise ohnehin vorhandenen Stapelzentriereinrichtungen erfolgen.

Die Notfallkofferhälfte umfasst gemäß einem Zusatzaspekt eine Kofferschale und eine Handhabeeinrichtung sowie einen hintergreifbaren Körper, wobei die Notfallkofferhälfte mit einer gleichartigen Kofferschale mit Handhabeeinrichtung und hintergreifbarem Körper verbindbar ist, um einen Notfallkoffer (insbesondere der oben beschriebenen Art in zumindest einer der oben beschriebenen Ausführungsformen, gegebenenfalls auch in einer der beschriebenen bevorzugten Ausführungsformen) bereitzustellen, bei dem durch jede Handhabeeinrichtung der einen Kofferschale der hintergreifbare Körper an der anderen Kofferschale hintergreifbar ist.

Gemäß Ausführungsformen weist die Notfallkofferhälfte einen Innendeckel auf. Die Kofferschale ist mit einer gleich gebauten Kofferschale verbindbar, insbesondere werkzeuglos verbindbar. Die genannte Notfallkofferhälfte lässt sich einzeln transportieren, etwa von zwei Bedienpersonen. Wird der Notfallkoffer nicht mehr benötigt, können sie zu einem Notfallkoffer zusammengesetzt werden.

Vorteilhafte Ausführungsformen des oben beschriebenen Notfallkoffers gemäß der Erfindung gelten naturgemäß auch für die Notfallkofferhälften, sofern anwendbar.

Insbesondere lässt sich die werkzeuglose Verbindbarkeit mit der genannten Stange und der dazu komplementären Steckvorrichtung an der Notfallkofferhälfte bewerkstelligen.

Ein Verfahren zum Verbinden zweier Notfallkofferhälften beinhaltet, dass die beiden Stangen der Notfallkofferhälften in die Steckvorrichtung der jeweils anderen Notfallkofferhälfte eingesteckt werden.

Eine Verwendung einer Notfallkofferhälfte ist eine Verwendung derselben einzeln als für sich tragbarer Notfallkoffer.

Gemäß einer weiteren bevorzugten Ausführungsform kann vorgesehen sein, dass der Notfallkoffer fluoresziert. Auf diese Weise ist er bei Nacht gut sichtbar, sodass Zeit bei der Bedienung eingespart werden kann.

Etwa kann der Notfallkoffer eine zumindest partiell fluoreszierend ausgebildete Bewandung haben. Das ist bei Verwendung von Kunststoff erzielbar, indem etwa dem Kunststoffgranulat vor einem Spritzgießen oder sonstiger Formgebung ein fluoreszierender Stoff beigefügt wird. Es kann sich beispielsweise um einen photolumineszierenden Stoff, insbesondere einen photolumineszierenden Stoff mit langer Nachleuchtzeit handeln, mit dem beispielsweise eine Leuchtdichte von 55/8 mcd/qm gemäß DIN 67510 erzielbar ist. Beispielsweise kann es sich bei dem fluoreszierenden Stoff um den aus EP 0 853 112 A1 an sich bekannten Stoff handeln; insbesondere kann der Stoff fluoreszierende Partikel beinhalten, insbesondere mit upconversion oder downconversion. In diesem Fall leuchten die Partikel auch dann, wenn über längere Zeit eine Anregung mit dem Umgebungslicht unterbleibt, beispielsweise im Falle eines Stromausfalls. Reststrahlung in einem anderen Frequenzbereich wird dann durch upconversion oder downconversion von diesen Partikeln im sichtbaren Bereich abgegeben, sodass der Notfallkoffer auch in diesem Fall gut sichtbar bleibt.

Alternativ oder zusätzlich können die Bewandungen des Notfallkoffers fluoreszierend bedruckt und/ oder mit fluoreszierender Folie beklebt werden.

Weiterhin ist es möglich, den Griff fluoreszieren zu lassen. Es kann also ein zumindest partiell fluoreszierend ausgebildeter und/ oder fluoreszierend bedruckter und/ oder mit fluoreszierender Folie beklebter Griff bereitgestellt werden. Dies lässt sich insbesondere an einem Griffeinsatz verwirklichen. Dann muss der restliche Griff nicht, und ggf. auch nicht der restliche Notfallkoffer, fluoreszierend ausgebildet sein, was die Herstellung preisgünstiger macht.

Dies kann beinhalten, dass der Griff alleine leuchtet. Dann ist der Griff besonders gut sichtbar, was bei Dunkelheit ein Ergreifen vor dem Heranziehen erleichtert. Dieser Effekt kann bei fluoreszierender Bewandung ebenfalls erzielt werden, indem der Griff heller fluoresziert als die Bewandung. Das Material für den Griff kann einen höheren Anteil an fluoreszierenden Partikeln aufweisen als der Notfallkoffer im übrigen.

Nachfolgend wird die Erfindung unter Bezug auf die Zeichnung näher beschrieben, in der
- Fig. 1: eine perspektivische Ansicht einer ersten Ausführungsform des erfindungsgemäßen Notfallkoffers zeigt;
- Fig. 2: eine Vorderansicht des Notfallkoffers aus Fig. 1 zeigt;
- Fig. 3: eine Draufsicht des Notfallkoffers aus Fig. 1 zeigt;
- Fig. 4: eine Unteransicht des Notfallkoffers aus Fig. 1 zeigt;
- Fig. 5: eine Seitenansicht des Notfallkoffers aus Fig. 1 zeigt;
- Fig. 6a: den Notfallkoffer aus Fig. 1 zeigt, wenn er geöffnet ist,
- Fig. 6b: dies weiter veranschaulicht, wenn die beiden Innendeckel geöffnet sind,
- Fig. 6c: dies weiter veranschaulicht, wenn die beiden Innendeckel zur einen Seite herumgeklappt sind;
- Fig. 6d: eine Notfallkofferhälfte des Notfallkoffers aus Fig. 1 im geöffneten Zustand mit darin befindlichen Trennelementen zeigt;
- Fig. 6e-6h: Trennelemente in verschiedenen Größen darstellen;
- Fig. 6i: das Trennelement aus Fig. 6f in vergrößerter Darstellung zur Veranschaulichung der Steckeinrichtungen der Trennelemente zeigt;
- Fig. 6k: eine Serie von Notfallkofferhälften unterschiedlicher Größen mit ihrer Facheinteilung in Draufsicht zeigt;
- Fig. 7a: eine zweite Ausführungsform des erfindungsgemäßen Notfallkoffers zeigt, in perspektivischer Ansicht von einer ersten Seite;
- Fig. 7b: diesen von einer zweiten Seite zeigt;
- Fig. 8: eine Vorderansicht des Notfallkoffers aus Fig. 7a zeigt;
- Fig. 9a und Fig. 9b: zwei verschiedene Seitenansichten des Notfallkoffers aus Fig 7a zeigen;
- Fig. 10: eine Draufsicht des Notfallkoffers aus Fig. 7a zeigt;
- Fig. 11a bis 11c: den Notfallkoffer aus Fig. 7a in Darstellungen entsprechend den Figuren 6a bis 6c veranschaulichen;
- Fig. 12a: eine Notfallkofferhälfte als Notfallkoffer gemäß einer dritten Ausführungsform der Erfindung veranschaulicht in perspektivischer Ansicht von einer ersten Seite;
- Fig. 12b: diesen von einer zweiten Seite veranschaulicht;
- Fig. 12c: die Situation beim Zusammenfügen zweier Notfallkofferhälften veranschaulicht;
- Fig. 12d: eine schematische Querschnittszeichnung zur Veranschaulichung des verwendeten Klemmprinzips ist;
- Fig. 12e: eine Kofferschale ohne Griff im geschlossenen Zustand in perspektivischer Ansicht veranschaulicht;
- Fig. 12f: die Kofferschale aus Fig. 12e im geöffneten Zustand in perspektivischer Ansicht veranschaulicht;
- Fig. 13: ein Stapelzentrierelement an der linken oberen Außenecke des Notfallkoffers aus Fig. 7 zeigt;
- Fig. 14: einen Stapel von mehreren Notfallkoffern zeigt;
- Fig. 15a: einen Ausschnitt aus Fig. 1 mit dem Griff zeigt;
- Fig. 15b: einen Ausschnitt aus Fig. 6a mit dem Griff zeigt;
- Fig. 15c: den Grundkörper des Griffs aus Fig. 15b alleine ohne das Einsatzteil zeigt;
- Fig. 15d: das in den Grundkörper einsteckbare Einsatzteil (Verschlusskappe) einzeln zeigt;
- Fig. 15e: den vollständigen Griff alleine mit dem Grundkörper aus Fig. 15c und mit dem darin eingesteckten Einsatzteil aus Fig. 15d zeigt;
- Fig. 15f: einen Schnitt durch den Notfallkoffer zur Sichtbarmachung eines in seinem Griff befindlichen Elektronikmoduls zeigt;
- Fig. 15g: den kreisförmigen Ausschnitt aus Fig. 15f in Vergrößerung zeigt;
- Fig. 16a: die Situation, in der ein Ersthelfer den auf dem Boden stehenden Koffer ergreift, zeigt;
- Fig. 16b: die Situation, in der ein Ersthelfer den auf dem Boden liegenden Koffer ergreift, zeigt;
- Fig. 17a: den auf dem Boden liegenden Koffer mit einem Griff im Zustand des Öffnens der Handhabeeinrichtungen zeigt;
- Fig. 17b: den kreisförmigen Ausschnitt aus Fig. 17a in Vergrößerung zeigt;
- Fig. 17c: eine Darstellung gemäß Fig. 17a im seitlichen Schnitt zeigt;
- Fig. 17d: eine perspektivische Ansicht auf den Notfallkoffer mit einem Griff von schräg vorne bei geschlossener Handhabeeinrichtung zeigt;
- Fig. 17e: den Koffer aus Fig. 17d mit geöffneter Handhabeeinrichtung zeigt;
- Fig. 17f: den Koffer aus Fig. 17d und Fig. 17e mit den Handhabeeinrichtungen in einer Parkstellung zeigt;
- Fig. 18a: eine Vorderansicht der Wandhalterung, die mit dem Notfallkoffer
- Fig. 18b: verwendbar ist in einer Version ohne Schloss zeigt; eine Seitenansicht der Wandhalterung aus Fig. 18a zeigt;
- Fig. 18c: eine perspektivische Ansicht der Wandhalterung aus Fig. 18a seitlich von vorne zeigt;
- Fig. 18d, 18e und 18f: den Fig. 18a, 18b bzw. 18c entsprechende Darstellungen der gleichen Art von Wandhalterung, nur mit einem Verriegelungsschloss versehen, zeigen;
- Fig. 18g: die Wandhalterung ohne Schloss mit daran aufgehängtem Notfallkoffer im geschlossenen Zustand zeigt;
- Fig. 18h: die Wandhalterung aus Fig. 18g mit dem Notfallkoffer im nunmehr geöffneten Zustand zeigt,
- Fig. 18i und 18k: eine Vorderansicht bzw. eine Seitenansicht der Wandhalterung mit dem geöffneten Notfallkoffer aus Fig. 18h zeigen; und
- Fig. 18l, 18m, 18n und 18o: den Fig. 18g, 18h Fig. 18i bzw. 18k entsprechende Darstellungen derselben Wandhalterung, nur mit Schloss, zeigen.

Ein im Ganzen mit 100 bezeichneter Notfallkoffer umfasst zwei Kofferschalen 10, die vorliegend gleichartig sind und im Falle des Notfallkoffers 100 jeweils gleichartige Griffe 12 tragen. Die Kofferschalen 10 mit den Griffen 12 bilden zusammen eine Notfallkofferhälfte, wobei im Falle des Notfallkoffers 100 zwei Notfallkofferhälften exakt zueinander komplementär sein können.

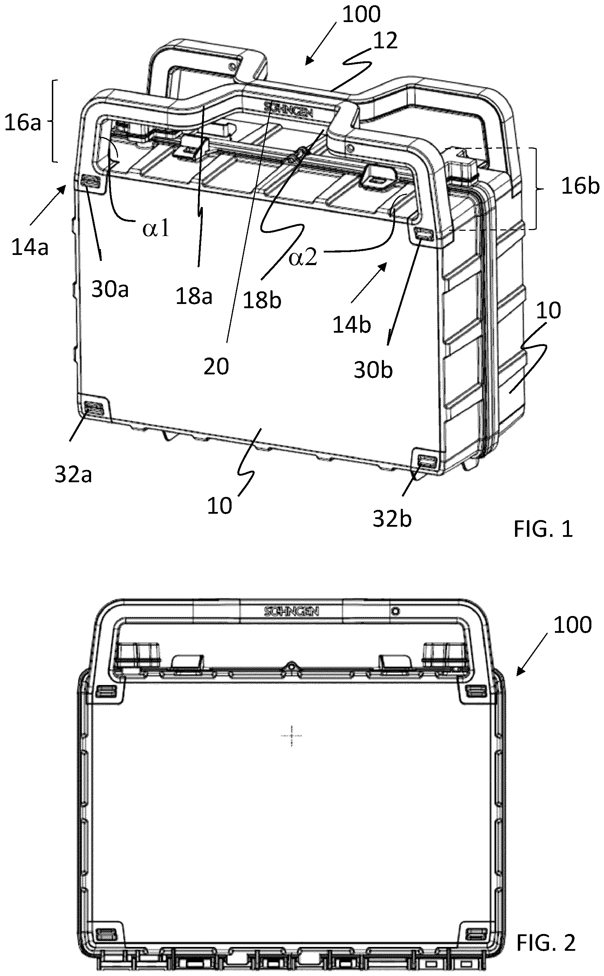

Die Griffe sind an den oberen Außenecken 14a, 14b jeder Kofferschale 10 befestigt, d.h. ein erstes Ende des Griffs 12 ist an der ersten oberen Außenecke 14a eingesteckt und ein zweites Ende des Griffs 12 an der anderen oberen Außenecke 14b der Kofferschale 10 eingesteckt. In einem ersten Abschnitt ausgehend von der jeweiligen oberen Außenecke erstreckt sich der Griff 12 vertikal nach oben, vorliegend sind dies die in Fig. 1 zu sehenden Abschnitte 16a (erster Abschnitt an dem ersten Griffende) und 16b (erster Abschnitt am zweiten Griffende). Unter "vertikal nach oben" wird verstanden, dass der Anstellwinkel a1 am ersten Griffende und der Anstellwinkel a2 am zweiten Griffende jeweils zwischen 70° und 110° liegt, vorzugsweise kleiner als 90° ist. Vorliegend sind beide Anstellwinkel a1 und a2 vorzugsweise gleich und haben hierzu beispielsweise einen Wert von ca. 75°.

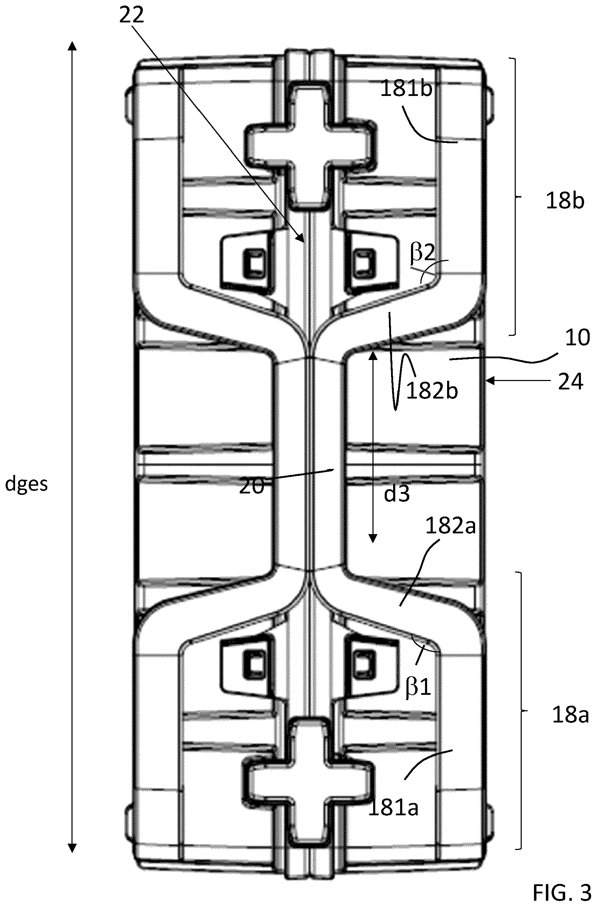

Aus der Draufsicht gemäß Fig. 3 ist erkennbar, wie sich der Griff 12 fortsetzt: In einem zweiten Abschnitt 18a bzw. 18b erstreckt sich jeder Griff zu der Innenlängskante 22 der Kofferschale hin. Vorliegend beinhaltet der zweite Abschnitt zwei Teilabschnitte, nämlich der Abschnitt 18a den Abschnitt 181a, der noch parallel zur Außenlängskante 24 der Kofferschale 110 verläuft, und den Abschnitt 182a, der von der Außenlängskante 24 hin zur Innenlängskante 22 verläuft. Der Winkel β1 der Anstellung des zweiten Teilabschnitts 182a gegenüber dem ersten Teilabschnitt 181a beträgt zwischen 10 und 20°, vorliegend ca. 15°. Der zweite Abschnitt 18b ist in analoger Weise in die Teilabschnitte 181b und 182b unterteilt, bei denen der Winkel β2 vorzugsweise ebenfalls zwischen 10 und 20° liegt und vorzugsweise denselben Wert wie der Winkel β1 hat.

Am Ende des zweiten Abschnitts 18b, insbesondere am Ende dessen zweiten Teilabschnitts 182b im Bereich der Innenlängskante 22 erstreckt sich der dritte Abschnitt 20 des Griffs 12. Dieser mittige Abschnitt erstreckt sich insbesondere parallel zur Innenlängskante 22, und bei dem Notfallkoffer 100 berühren sich der dritte Abschnitt 20 der ersten Notfallkofferhälfte an der ersten Kofferschale 10 und der dritte Abschnitt 20 der entsprechenden zweiten Notfallkofferhälfte unmittelbar oberhalb der Innenlängskante 22. Die Länge d3 des dritten Abschnitts 20 beträgt nahezu ein Drittel der Gesamtlänge des gesamten Koffers und damit des gesamten Griffs 12, wobei die Abschnitte 18b und 18a das andere Drittel ergänzen, bzw. geringfügig länger als das Drittel sind. Durch diese Abmessungen ist für eine Austarierung des Koffers gesorgt, wenn die Bedienperson den Koffer an den beiden dritten Abschnitten 20 umgreift.

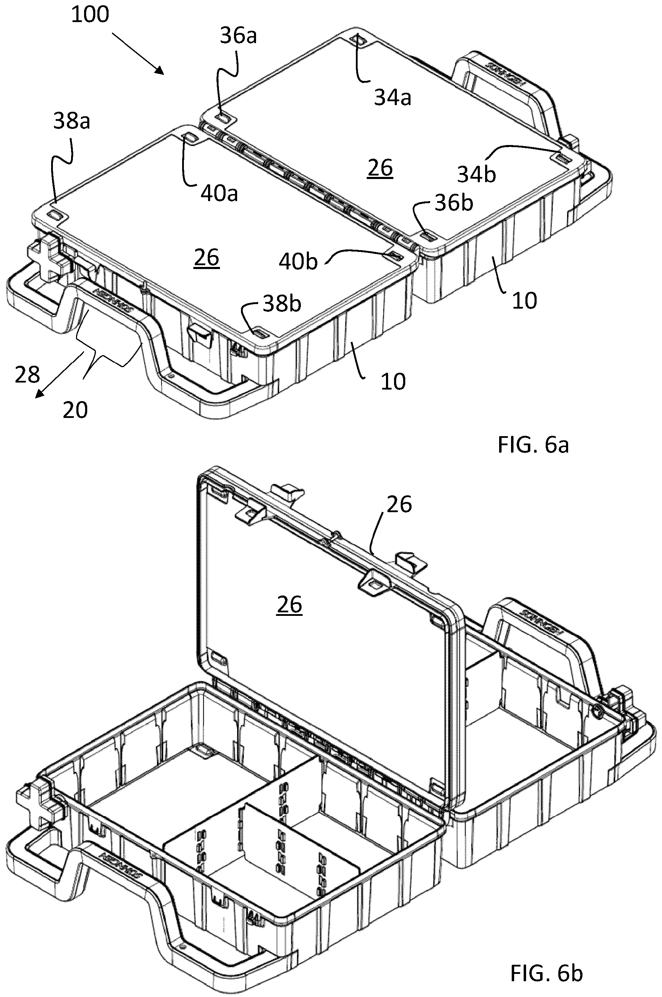

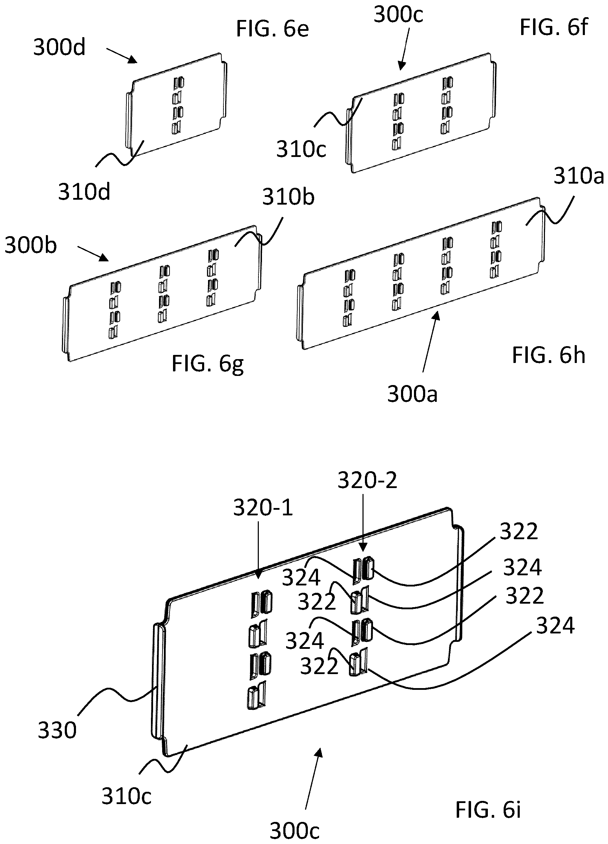

Die Koffer lassen sich gemäß Fig. 6a aufklappen, wodurch sichtbar wird, dass es einen vorzugsweisen opaken Innendeckel 26 an der Kofferschale 10 gibt. Der aufgeklappte Notfallkoffer lässt sich an dem beim Liegen nach oben hochstehenden dritten Abschnitt optimal ergreifen, so dass für die Bedienung in einer Notfallsituation am Patienten vor Ort ein schnelles Heranziehen des Koffers ermöglicht ist, etwa in die Richtung gemäß dem Pfeil 28 in Fig. 6a.

Gemäß Fig. 6b lassen sich die beiden Innendeckel 26 hochklappen und komplementär zueinander senkrecht hochstellen.

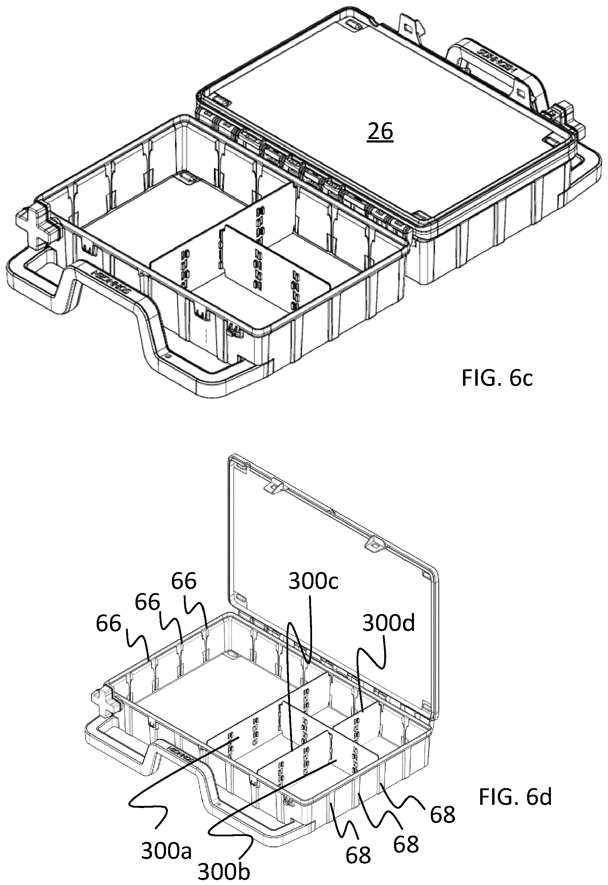

Gemäß Fig. 6c lassen sich die beiden miteinander verrasteten Deckel auch nur zu einer 5 Seite hinklappen, vorliegend der hinteren Seite in Fig. 6c.

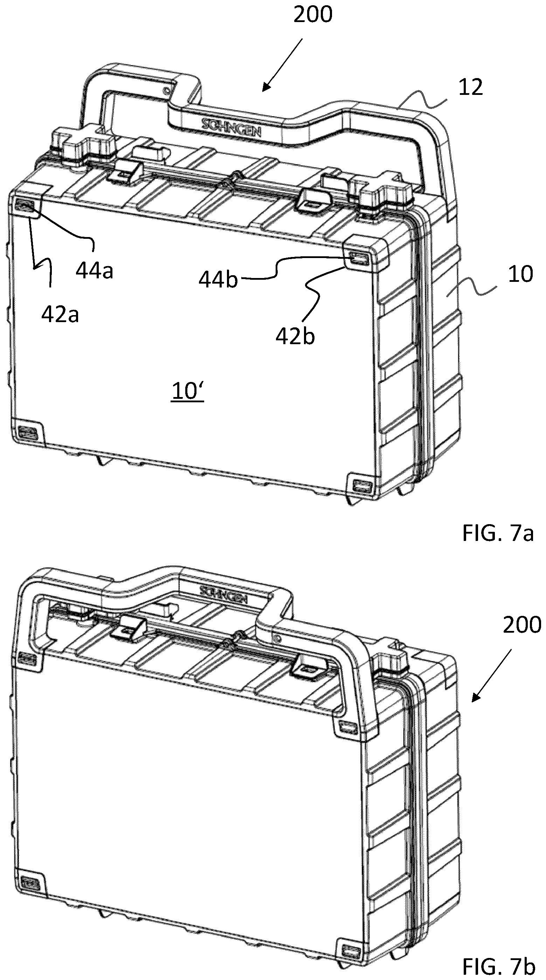

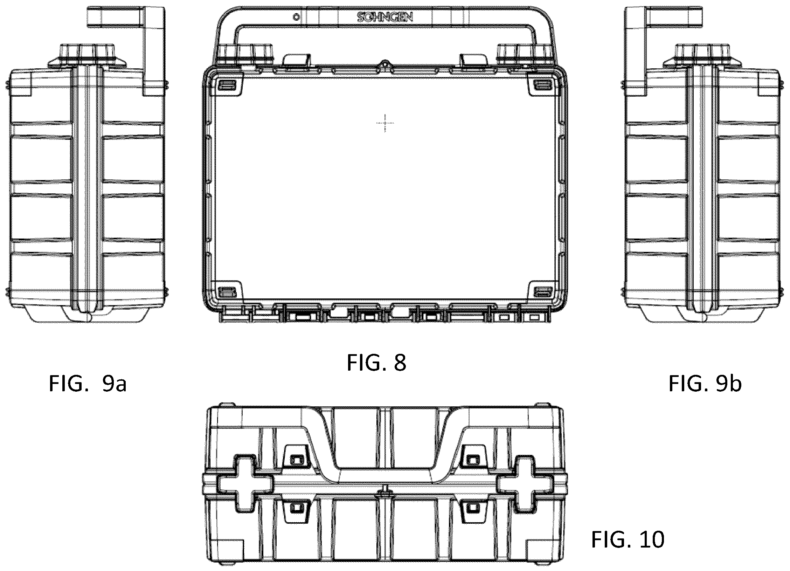

Die nachfolgend erläuterte zweite Ausführungsform 200 des Notfallkoffers gemäß Fig. 7a bis Fig. 11c unterscheidet sich von der bisher beschriebenen Ausführungsform 100 des Notfallkoffers darin, dass an nur einer Kofferschale 10 der Griff 12 ausgebildet ist, wohingegen an der anderen Kofferschale 10' an den oberen Außenecken Blindkappen 42a, 42b eingebracht sind. Die Blindkappen ersetzen den Griff bei der Kofferschale 10'.

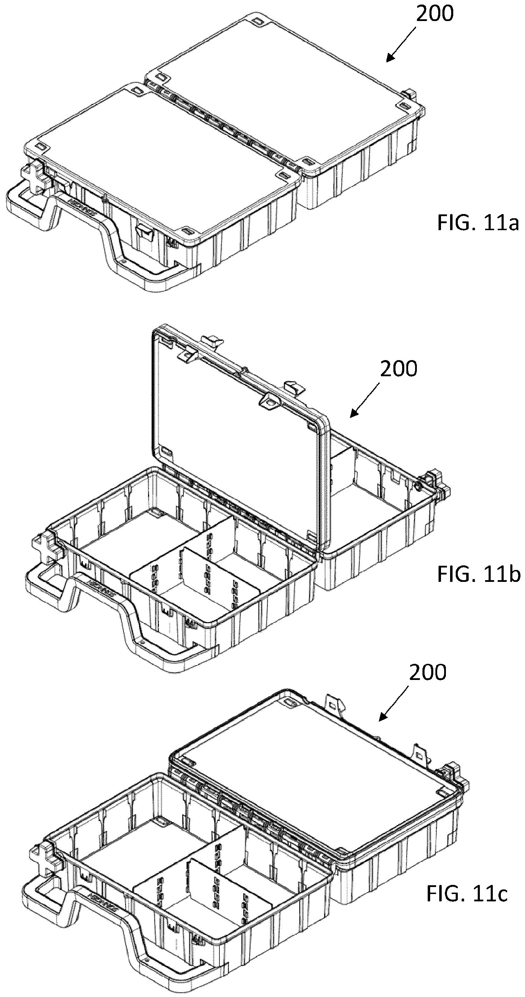

Durch das Entfallen eines Griffs wird der Notfallkoffer 200 leichter als der Notfallkoffer 100. Die Tragbarkeit ist durch die Form des Griffes allerdings nach wie vor sehr erleichtert. Die Figuren 11a bis 11c entsprechen den Figuren 6a bis 6c.

Damit die Notfallkoffer mit anderen Notfallkoffern (etwa in einem Krankenwagen) geeignet stapelbar sind, gibt es sogenannte Stapelzentriereinrichtungen. Solche Einrichtungen sind in der Regel paarweise zueinander komplementär (auf Umschlag).

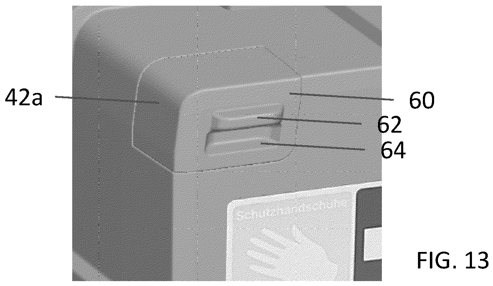

Fig. 13 veranschaulicht eine Stapelzentriereinrichtung an einer linken oberen Außenecke des Notfallkoffers 200, also an der Blindkappe 42a. Die diesbezügliche Beschreibung einer beispielhaften Ausgestaltung gilt für alle Stapelzentriereinrichtungen. Ausgehend von einer glatten, ebenen Oberfläche, die sich an die Oberfläche außerhalb der Blindkappe 42a anschließt, gibt es einen Vorsprung 62, hier mit einer Längserstreckung parallel zur Oberkante der Kofferschale 10. Unterhalb des Vorsprungs 62 gibt es eine Ausnehmung 64, die in der Form komplementär zum Vorsprung 62 ist. Die Rollen von Vorsprung und Ausnehmung sind an den auf Umschlag vorgesehenen Stapelzentriereinrichtungen vertauscht. Etwa an der rechten oberen Außenecke ist dort an der Stapelzentriereinrichtung 42b eine Ausnehmung oben und ein Vorsprung darunter vorgesehen. So passt der Vorsprung einer Kofferschale in die Ausnehmung an einer anderen eines anderen Notfallkoffers.

Im Einzelnen zu den Stapelzentriereinrichtungen: Beim Notfallkoffer 100 sind an den Griffenden an den oberen Außenecken der Kofferschale 10 die Stapelzentriereinrichtungen 30a und 30b vorgesehen, die zueinander auf Umschlag sind. An den unteren Ecken sind ihrerseits Stapelzentriereinrichtungen 32a, 32b ausgebildet, wobei die Stapelzentriereinrichtung 32a mit Stapelzentriereinrichtung 32b komplementär ist. Die Stapelzentriereinrichtung 32a ist ferner mit der Stapelzentriereinrichtung 30a komplementär, die Stapelzentriereinrichtung 32b mit der Stapelzentriereinrichtung 30b komplementär (auf Umschlag).

Damit auch geöffnete Notfallkoffer stapelbar sind, weisen ferner die Innendeckel 26 Stapelzentriereinrichtungen auf, siehe an dem einen Innendeckel die Bezugszeichen 34a, 34b und 36a und 36b. Die dazu komplementären Stapelzentriereinrichtungen 38a und 38b an der anderen Kofferhälfte und 40a, 40b, sind paarweise komplementär zu den Stapelzentriereinrichtungen: 38a zu 34a, 38b zu 34b, 40a zu 36a und 40b zu 36b. Auf diese Weise lassen sich die beiden Innendeckel 26 auch gut miteinander verrasten, wie in Fig. 6b gezeigt.

Bei den Notfallkoffern, mit und ohne Griff weist jede Kofferschale Trennelemente zur Bildung von Fächern auf. Eine geöffnete Notfallkofferhälfte ist in Fig. 6d gezeigt. Die darin verwendeten Trennelemente sind einzeln in den Figuren 6e bis 6h gezeigt, das Trennelement aus Fig. 6f in Vergrößerung in Fig. 6i.

Vorliegend ist, wie aus Fig. 6d ersichtlich, an allen vier Innenwänden der rechteckigen Kofferschale jeweils eine Folge von Steckeinrichtungen 66 bereitgestellt. Die Steckeinrichtungen 66 haben schwalbenschwanzförmige Einlässe, in die die Trennelemente 300a, 300b, 300c, 300d jeweils einsteckbar sind. Wie aus den Figuren 6e bis 6h ersichtlich, umfasst jedes der Trennelemente 300a, 300b, 300c, 300d einen streifenförmigen Grundkörper 310a, 310b, 310c, bzw. 310d. An jedem streifenförmigen Grundkörper gibt es seinerseits Steckeinrichtungen; in Fig. 6i wird dies insbesondere noch veranschaulicht für das Trennelement 300c, welches genau zwei solcher Steckeinrichtungen 320-1 und 320-2 aufweist. Jede Steckeinrichtung 320-1, 320-2 umfasst mehrere Nasen, wie vorliegend anhand der Steckeinrichtung 320-2 zu sehen: Die Nasen 322 alternieren rechts und links untereinander, wobei jeder Nase 322 eine Aussparung 324 gegenüberliegt, welche nichts anderes als eine Ausstülpung ist, der auf der Fig. 6i zu denkenden Rückseite genau wieder einer Nase entspricht. Somit alternieren die Aussparungen links und rechts untereinander ebenfalls.

Jedes Trennelement weist eine Schiebeschiene 330 auf. Diese ist in die schwalbenschwanzförmigen Aussparungen der Steckeinrichtung 66 einschiebbar und so in dieser unmittelbar feststeckbar. Durch die unmittelbare Feststeckbarkeit an den Innenwänden der Kofferschale ist für eine besonders stabile Anordnung gesorgt. Damit die Steckeinrichtung 66 die Kofferschalenwand nicht schwächen, gibt es ihnen entsprechend außengenau Verstärkungsrippen 68, also Verdickungen oder Ausstülpungen der Außenwand.

Die Trennelemente 300a, 300b, 300c, 300d haben die Längen als gemeinsame Vielfache einer Grundeinheit, nämlich das Trennelement 300a mit fünf Grundeinheiten, das Trennelement 300b mit vier Grundeinheiten, das Trennelement 300c mit drei Grundeinheiten und das Trennelement 300d mit zwei Grundeinheiten. So ist die Facheinteilung gemäß Fig. 6d erzielbar mit dem Trennelement 300a, dem Trennelement 300b, dem Trennelement 300c und dem Trennelement 300d, jeweils in einfacher Verwendung. Hier ist das Trennelement 300a in einander gegenüberliegende Steckeinrichtungen an den Innenwänden eingesteckt, das Trennelement 300b ist an einer Steckeinrichtung am Trennelement 300a einerseits und einer Steckeinrichtung an der anderen Innenwand der Notfallkofferhälfte eingesteckt. Das Trennelement 300c ist mit einer Schiebeschiene an der mittleren Steckeinrichtung des Trennelements 300b eingesteckt und mit der anderen Schiebeschiene an einer Steckeinrichtung an der vorderen Innenwand der Notfallkofferhälfte eingesteckt, und schließlich ist das Trennelement 300d mit einer Schiebeschiene an der mittleren Steckeinrichtung auf der anderen Seite des Trennelements 300b als das Trennelement 300c eingesteckt, und mit der anderen Schiebeschiene ist das Trennelement 300d an einer Steckeinrichtung 66 an der hinteren Innenwand der Notfallkofferhälfte eingesteckt.

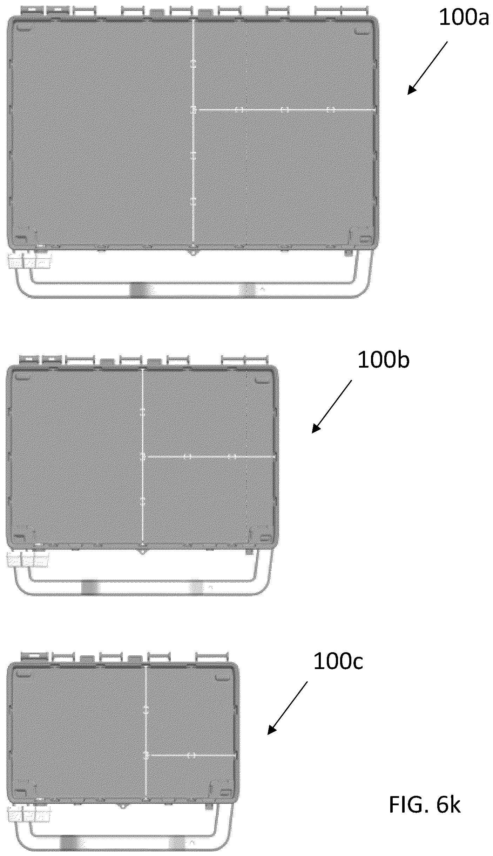

Fig. 6k zeigt, dass durch Wahl der geeigneten Größenverhältnisse in einer Serie von Notfallkoffern 100a, 100b und 100c verschiedener Größen die genannten Trennelemente immer wieder neu einsetzbar sind. Der obere Notfallfallkoffer 100a hat in einer Abmessungsrichtung bei gleicher Höhe eine Erstreckung von fünf Einheiten, der mittlere Notfallkoffer 100b eine Erstreckung von vier Einheiten und der untere, kleinste Notfallkoffer eine Erstreckung von drei Einheiten. Somit können in den Notfallkoffern immer wieder die gleichen Trennelemente verwendet werden, was die Herstellung unterschiedlicher Serien vereinfacht.

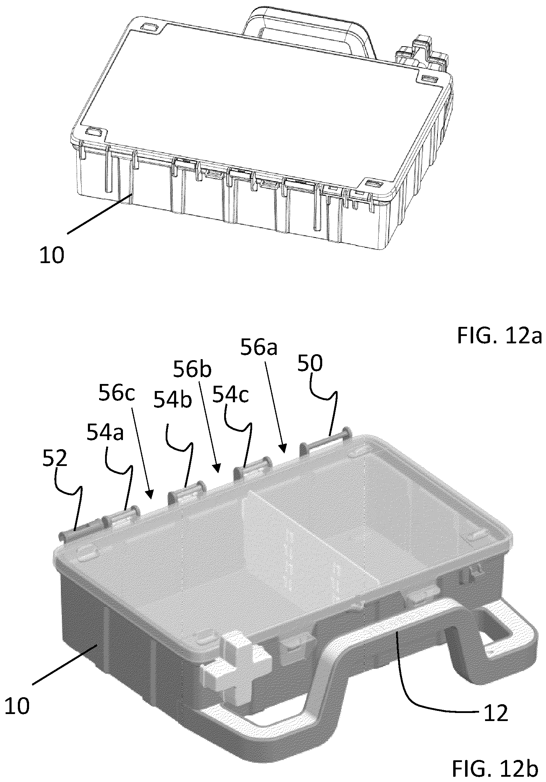

Die beiden Notfallkofferhälften mit den Kofferschalen 10 und dem Griff 12 bei dem Notfallkoffer 100 aus Fig. 1 bis 6c bzw. die entsprechende Kofferhälfte des Notfallkoffers 200 lässt sich auch einzeln verwenden, wie in Fig. 12a und 12b veranschaulicht. Insbesondere sind die beiden Notfallkofferhälften bei den Notfallkoffern 100 und 200 voneinander lösbar. Am unteren Bereich der jeweiligen Notfallkofferhälfte sind zueinander komplementäre Steckvorrichtungen vorgesehen: Eine Stange 50 am unteren Bereich der Notfallkofferhälfte mit der Schale 10 und dem Griff 12 lässt sich in die im Querschnitt C-förmige elastische Steckvorrichtung 52 einer gleich gebauten Notfallkofferhälfte einstecken. Entlang der Achse befinden sich ferner die Einrichtungen 54a, 54b und 54c zur Befestigung des Innendeckels. Es handelt sich hierbei um Scharniere, wobei die Einrichtungen 54a, 54b und 54c beispielsweise jeweils auch eine Stange aufweisen können, an die ein C-förmiges elastisches Teil, das zum Innendeckel gehörig ist, anklemmen lässt. So lässt sich der Innendeckel gegebenenfalls auch abnehmen. Die entsprechenden Einrichtungen 54a, 54b und 54c lassen sich in die Lücken 56a, 56b, 56c der komplementären Notfallkofferhälfte einfügen, so dass eine geschlossene Drehachse gebildet ist.

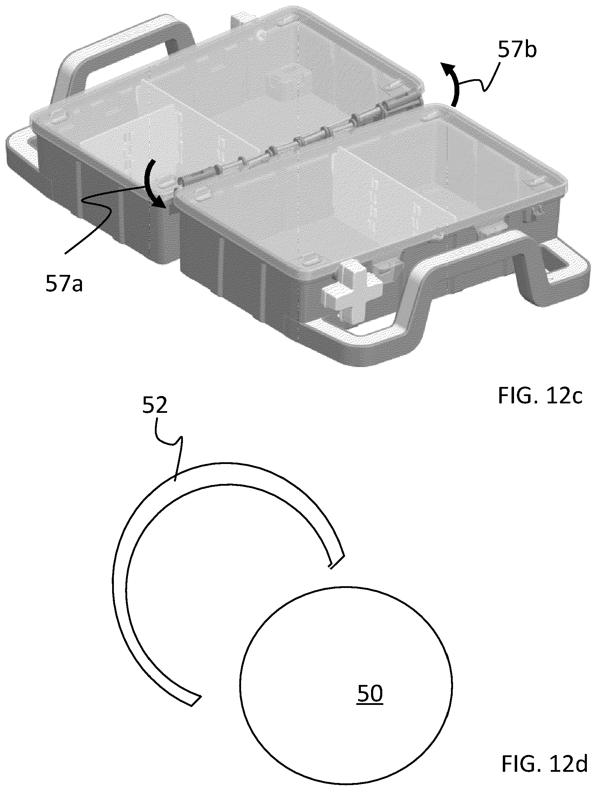

Die Fig. 12c veranschaulicht die Situation, in der gerade die beiden Notfallkofferhälften 10 miteinander verbunden werden: Hier wird die Stange 50 der einen Notfallkofferhälfte in die Steckvorrichtung 52 der gleichgebauten anderen Notfallkofferhälfte eingesteckt (siehe Pfeil 57a) und entsprechend die Stange 50 der anderen Notfallkofferhälfte in die Steckvorrichtung 52 der einen Notfallkofferhälfte eingesteckt (siehe Pfeil 57b).

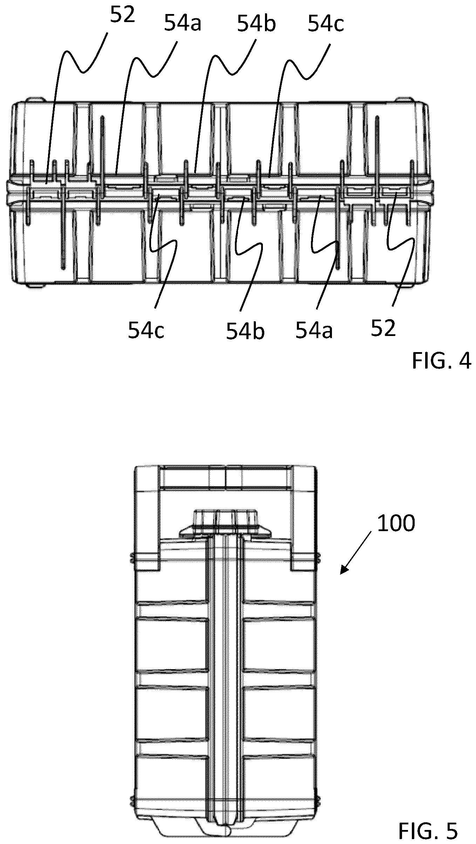

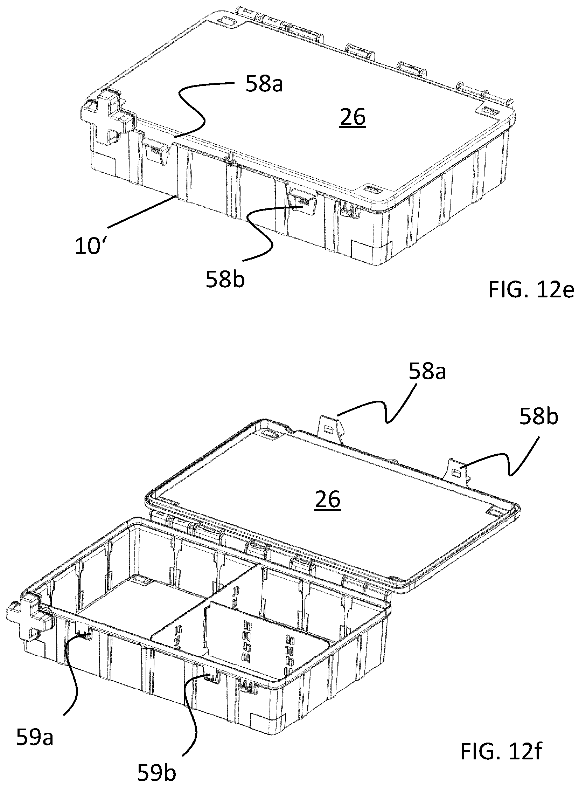

Aus Fig. 4 ist ersichtlich, wie der so zusammengesteckte Notfallkoffer 100 von unten aussieht. (Entsprechendes gilt für den Notfallkoffer 200.): Die Lücken 56a, 56b, 56c sind nicht mehr zu sehen, sondern hier sind die Scharniere 54a, 54b, 54c, an die entsprechende C-förmige elastische Steckelemente des Innendeckels 26 angebracht sind, der komplementären Notfallkofferhälfte jeweils eingefügt. Die Fig. 12d zeigt im Querschnitt die Stange 50 neben der Steckvorrichtung 52. Unter Auffedern der Steckvorrichtung 52 lässt sich die Stange in diese Hineinstecken, nach dem Zurückfedern ist sie darin gehalten. Die Fig. 12e und 12f zeigen eine Kofferschale 10' ohne Griff. Auf diese ist bis auf die Lehre zum Griff anwendbar, was zur Notfallkofferhälfte beziehungsweise Kofferschale 10 hier ausgeführt wird. Auf die Kofferschale 10 ist anwendbar, was zur Kofferschale 10' angegeben wird, sofern nicht das Weglassen des Griffs betreffend.

Die Klemmlaschen 58a und 58b sind mit dem übrigen Abschnitt des Innendeckels 26 einstückig ausgebildet. Alternativ sind sie an einen Innendeckel angestückt. Der Klemmlasche 58a entspricht die Rastnase 59a, an der sie verrastbar ist, und der Klemmlasche 58b ent5 spricht die Rastnase 59b, an der sie verrastbar ist. So lässt sich der Innendeckel 26 fest schließen, um die jeweilige Kofferschale 10 oder auch 10' einzeln tragen zu können, ohne dass etwas herausfällt.

Der Innendeckel 26 ist an der Kofferschale 10 unter Einsatz des selben Klemmprinzips befestigt, wie oben unter Bezug auf die Fig 12d beschrieben: Im Bereich der Scharniere 54a, 54b und 54c ist jeweils an der Kofferschale 10 bzw. 10' eine Stange ausgebildet, an der eine C-förmige elastische Steckvorrichtung des Innendeckels festgeklemmt ist. Diese C-förmige elastische Steckvorrichtung sind alle mit dem übrigen Abschnitt des Innendeckels 26 einstückig ausgebildet. Alternativ sind sie an einen Innendeckel angestückt.

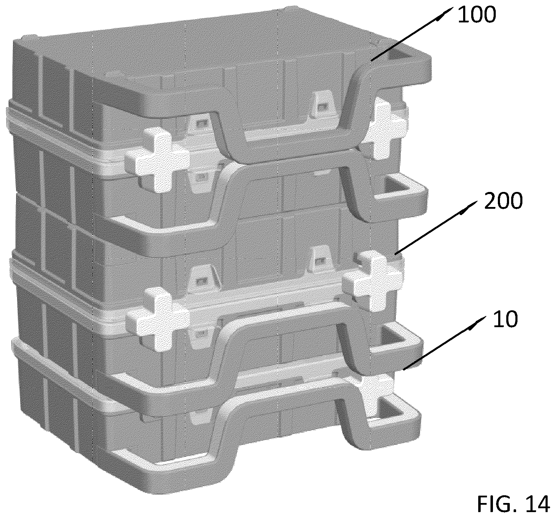

Fig. 14 zeigt einen Stapel aus a) dem Notfallkoffer wie in Fig 12a, b) dem Notfallkoffer 200 aus Fig. 7a und dem Notfallkoffer 100 aus Fig 1. Zur Stabilität tragen die Stapelzentriereinrichtungen bei.

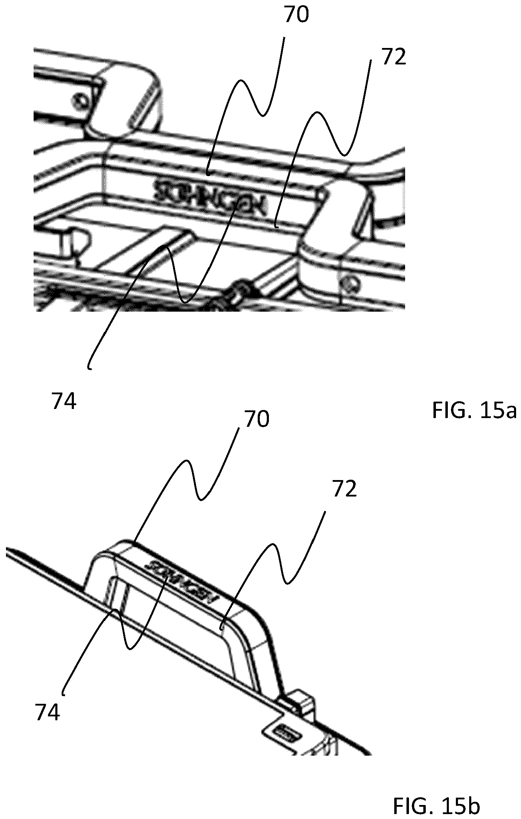

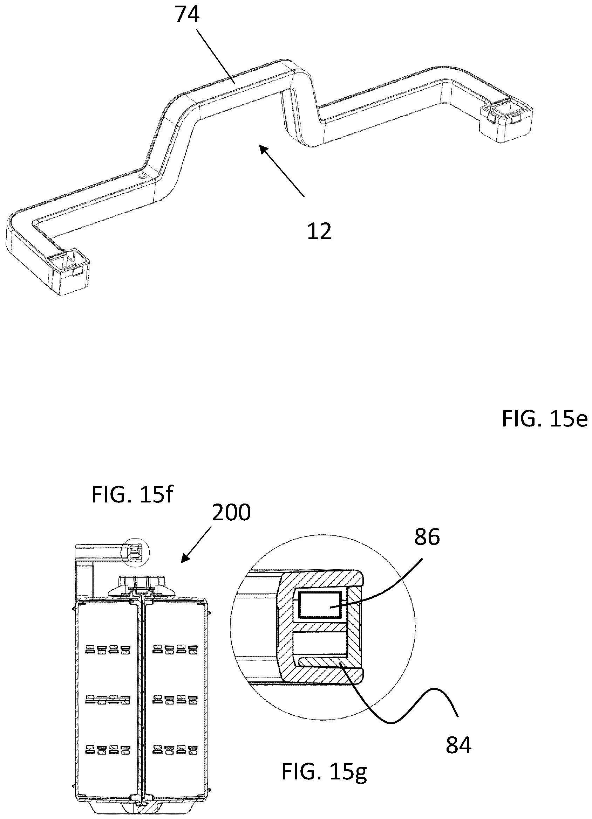

Fig. 15a zeigt einen Ausschnitt aus Fig. 1 mit dem Griff, wie er von außen zu sehen ist. Es ist daraus ersichtlich, dass der Griff einen Rahmen mit oberem Rahmenteil 70 und unterem Rahmenteil 72 aufweist. Zwischen die beiden Rahmenteile 70 und 72 ist ein Einsatzteil 74 des Griffs eingesetzt. Das Einsatzteil 74 kann eingeklebt oder eingepresst sein, ggf. auch mit im Innern des Griffs befindlichen Elementen verclipst sein.

Fig. 15b zeigt einen Ausschnitt aus Fig. 6a mit dem Griff, der darin mit seiner Innenseite sichtbar ist. Auch hier ist zwischen den Rahmenteilen 70 und 72 das Einsatzteil 74 zu sehen. Es kann sich um dasselbe Einsatzteil handeln wie bei Fig. 15a. Alternativ können zwei Einsatzteile vorgesehen sein (nicht dargestellt).

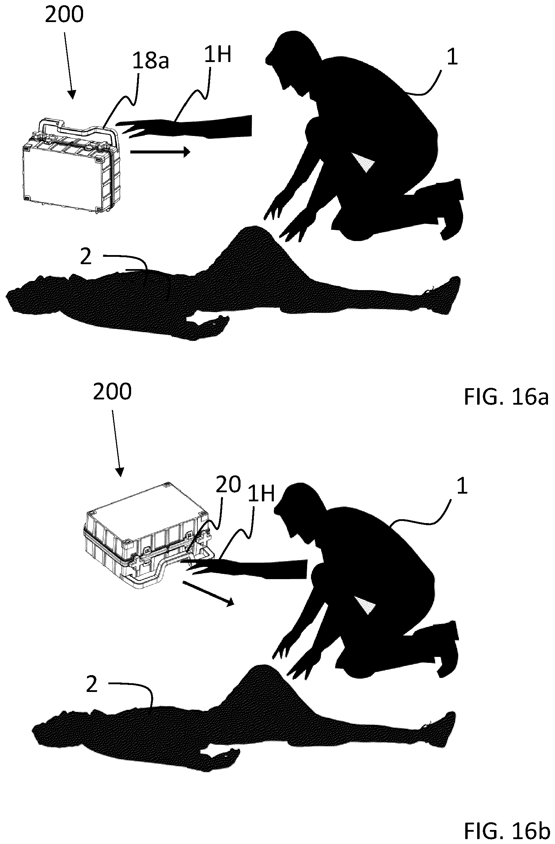

Fig. 16a veranschaulicht, wie ein Ersthelfer 1 bei einem Patienten 2 kniet. Der Notfallkoffer 200 mit einem Griff 12 lässt sich durch den Ersthelfer 1 mit seiner Hand 1H am Abschnitt 18c ergreifen, wenn er steht, und so weiter heranziehen.

Gemäß Fig. 16b zieht der Ersthelfer 1 mit seiner Hand 1H in der anderen Situation den dort liegenden Notfallkoffer 200 herbei, indem er ihn am Abschnitt 20 ergreift.

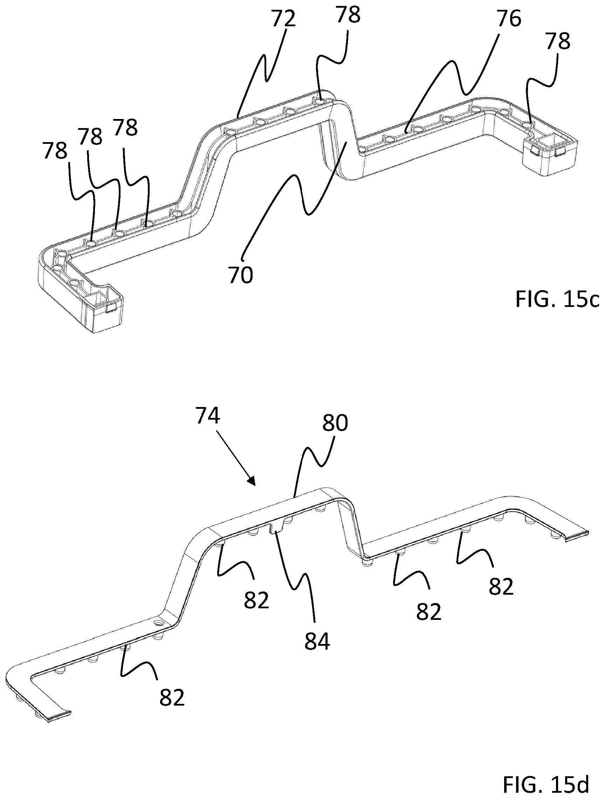

Fig. 15c zeigt den Griff aus Fig. 15b einzeln ohne das Einsatzteil, also lediglich den, vorzugsweise aus Kunststoff spritzgegossenen, Grundkörper des Griffs. Sichtbar ist eine Kavität 76 in dem Grundkörper. Diese ist nach einer Seite hin offen, nämlich vorliegend zur oberen Seite hin. In dem Grundkörper ist über die gesamte, oben beschriebene Erstreckung (erster Bereich, zweiter Bereich jeweils zweifach und dazwischenliegender dritter Bereich) eine Folge von kreisförmigen Aussparungen 78 vorgesehen.

Das Einsatzteil 74, das sich auch als Verschlusskappe bezeichnen lässt, ist im Ganzen in Fig. 15d einzeln gezeigt. Den kreisförmigen Aussparungen 78 am Grundkörper entsprechen Noppen 82, die sich in die Aussparungen einstecken lassen, wobei ein leichtes Verrasten erfolgt. Diese sind über den leistenförmigen Grundkörper 80 des Einsatzteils/der Verschlusskappe 74 verteilt. Mittig, dem dritten Bereich des Griffs entsprechend, gibt es eine Stecklasche 84.

Wie aus der Schnittansicht gemäß Fig. 15f zu sehen, sorgt die Stecklasche 84 für einen besonders guten Halt eines in die Kavität 76 des Grundkörpers dort eingebrachten Elektronikmoduls 86. Dieses kann beispielsweise einen Speicher aufweisen, in dem angegeben ist, welche Materialien in dem Notfallkoffer/Erste-Hilfe-Koffer vorhanden sein sollen bzw. müssen. Das Elektronikmodul 86 kann ein drahtloses Auslesen dieser Information ermöglichen und beispielsweise durch ein Abfragesignal Energie zum Aussenden eines Antwortsignals erhalten, über das diese Information nach außen gegeben wird.

Die Notfallkofferhälfte lässt sich einzeln tragen, auch weil der Innendeckel 26 geeignet an der Kofferschale 10 bzw. 10' verrastbar ist. Dies kann beispielsweise durch einen umlaufenden Steg an dem Innendeckel erfolgen, der den Rahmen der Kofferschale 10, 10' einfach durchgehend umgreift. Dadurch, dass der Innendeckel opak ist, wird das Material in dem Verbandskoffer wird vor Alterung durch Ultraviolettstrahlung geschützt. Auch können Dritte nicht ins Innere der Notfallkofferhälfte blicken, wenn diese einzeln getragen wird. Alternativ ist der Innendeckel transparent ausgestaltet.

Das Einsatzteil 74 kann fluoreszierend ausgebildet sein, beispielweise durch Beimengung fluoreszierender Partikeln, wie sie von der Fa. American Permalight Corp. unter dem Markennamen PERMALIGHT^{®} vertrieben werden. Das Einsatzteil trägt im Beispiel zudem ein Logo. Dadurch ist der Griff auch bei Nacht gut sichtbar, zum Beispiel für den vor dem Patienten knienden Arzt, der ihn beiziehen möchte.

Der Notfallkoffer kann im Übrigen in anderer Farbe als das Einsatzteil ausgebildet sein. Er kann auch seinerseits fluoreszierend sein, vorzugsweise schwächer fluoreszierend als das Einsatzteil des Griffs. Am einfachsten lässt sich eine fluoreszierende Folie aufbringen, dann kann ein strukturiertes Muster zum Leuchten gebracht werden.

Des Weiteren kann der Innendeckel 26 ebenfalls fluoreszierend sein, damit sich auch eine Notfallkofferhälfte einzeln besonders gut erkennen lässt.

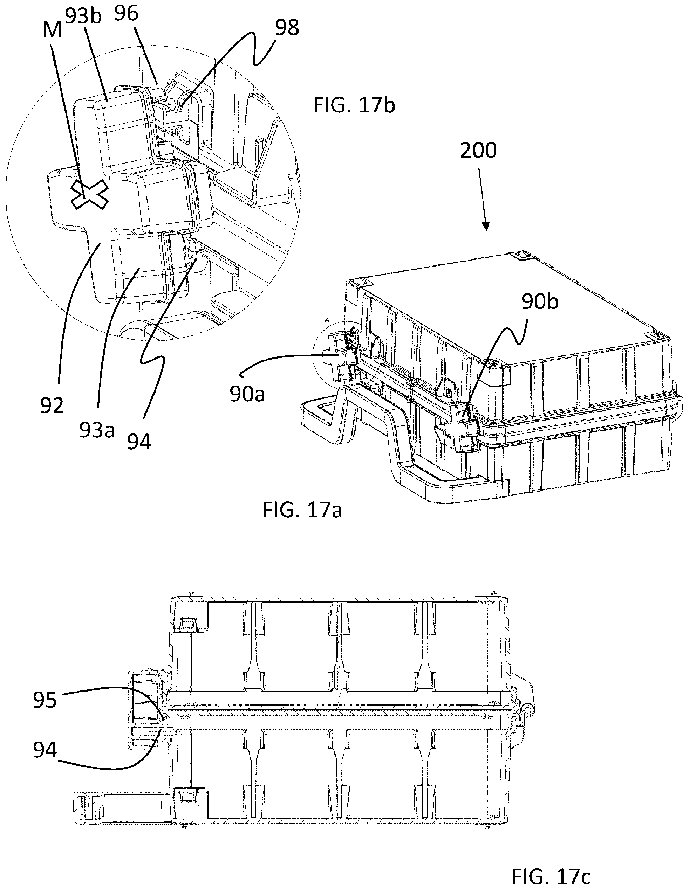

Das Öffnen und Schließen des Notfallkoffers ist besonders einfach aufgrund der Gestaltung der Handhabeeinrichtungen 90a, 90b möglich. Hierzu zeigt die Fig. 17a den Notfallkoffer 200 im Zustand, in dem die Handhabeeinrichtungen gerade geöffnet werden. Fig. 17b zeigt den kreisförmigen Ausschnitt aus Fig. 17a in Vergrößerung.

Es ist ersichtlich, dass jede Handhabeeinrichtung 90a wie 90b im Wesentlichen drei Komponenten umfasst, nämlich die eigentliche Handhabe 92, die vorliegend kreuzförmig ist. Die Kreuzform beinhaltet das Vorsehen gleich langer, in einem Winkel von 90° zueinander stehender Schenkel, von denen der untere Schenkel 93a und der obere Schenkel 93b einander gegenüberliegen. Die Handhabe 92 ist auf der Vorderseite der Kreuzform glatt und hat an jedem Schenkel im Wesentlichen senkrecht zur vorne glatten Oberfläche stehende Seitenwandungen.

Die zweite Komponente ist die an dem Schenkel 93a ausgebildete Hohlwelle 94, die in 5 einen Flansch 95 an der zugehörigen Kofferschale eingesteckt ist.

Die dritte Komponente der Handhabeeinrichtung 90a, 90b ist ein Hintergreifelement in Form einer Hintergreifnase 96, die man auch als Hintergreifhaken oder Hintergreifkralle bezeichnen kann. Diese Hintergreifnase 96 hintergreift beim Schließen die Rastnase 98 an der jeweils der Handhabeeinrichtung nicht zugehörigen anderen Kofferschale. Beim Aufliegen des Koffers ist die linke vordere Handhabeeinrichtung zur unteren Kofferschale gehörig, die rechte vordere Handhabeeinrichtung zur oberen Kofferschale gehörig. Entsprechend ist die Rastnase 98, die von der linken Handhabeeinrichtung 90a hintergriffen wird, an der oberen Kofferschale ausgebildet und eine entsprechende Rastnase 98, die von der rechten Handhabeeinrichtung 90b hintergriffen wird, an der unteren Kofferschale ausgebildet (wobei Letztere in der Zeichnung nicht dargestellt ist).

Zu den Rastnasen 98 ist noch anzuführen, dass diese bevorzugt wie in Fig. 17b ausgebildet sind: An der Kofferschale (in Fig. 17b der oberen Kofferschale) ist die Seitenwand verstärkt ("Bodenplatte" für die Rastnase), worauf dann auch noch ein Paar von Füßen steht, von denen (vorzugsweise im Wesentlichen senkrecht zur Oberfläche der Vorderwand der Kofferschale und damit der "Bodenplatte") ein kleiner Arm wegsteht, der die Rastnase trägt. Die Hintergreifnase 96 ist über den Arm schwenkbar und aufgrund der abgerundeten Ausbildung der Füße kann die Hintergreifnase 96 die andere (in Fig. 17b obere) Kofferschale ein wenig nach unten ziehen, so dass der Notfallkoffer abdichtend geschlossen ist.

Beachtenswert ist, dass die Kreuzform aufgrund der gleich langen Schenkel ihren geometrischen Mittelpunkt M genau in der Mitte, dem Schnittpunkt der beiden das Kreuz bildenden Linien, aufweist. Entsprechend der Anordnung der Hohlwelle 94 an einem der Schenkel ist die Handhabeeinrichtung also exzentrisch gelagert. Die exzentrische Lagerung hat den Vorteil, dass die Handhaben in einem wesentlich größeren Maße verschwenkbar sind, als sie es bei anderer Lagerung wären. Insbesonders sind sie überhaupt verschwenkbar und nicht nur drehbar.

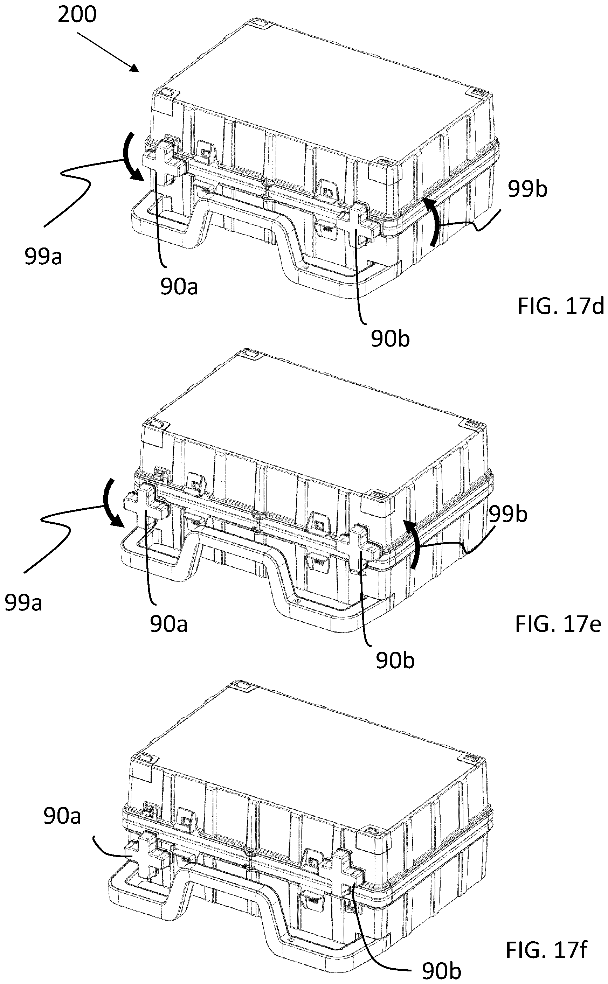

Fig. 17d zeigt den Notfallkoffer 200 im geschlossenen Zustand. Durch die Pfeile 99a und 99b ist dargestellt, in welcher Schwenkrichtung die Handhaben 92 der Handhabeeinrichtung 90a bzw. 90b verschwenkt werden müssen. Im Übergang zu Fig. 17e schwenkt die Hintergreifnase 96 hinter der Rastnase 98 hervor in die in Fig. 17e gezeigte Offenstellung. In letzterer lässt sich der Notfallkoffer bereits öffnen.

Gemäß weiterer Drehung in die Richtung gemäß dem Pfeil 99a und 99b lässt sich sodann auch noch eine Parkstellung, wie in Fig. 17f dargestellt, erzielen. Diese Parkstellung hat den Vorteil, dass sich der Notfallkoffer noch leichter öffnen lässt und insbesondere die Handhabeeinrichtungen weniger stören. Dies ist insbesondere dann hilfreich, wenn der Notfallkoffer 200, wie oben dargestellt, in zwei Notfallkofferhälften geteilt werden soll.

Im Übergang von Fig. 17d zu Fig. 17e, also von der Schließstellung in die Ruhestellung, er5 folgt eine Drehung (Verschwenkung) der Handhabe um 90°. Im weiteren Übergang zur Parkstellung gemäß Fig. 17f erfolgt eine weitere Drehung um weitere 90°. Insgesamt verschwenkt man von der Schließstellung in die Parkstellung die Handhabe um 180°.

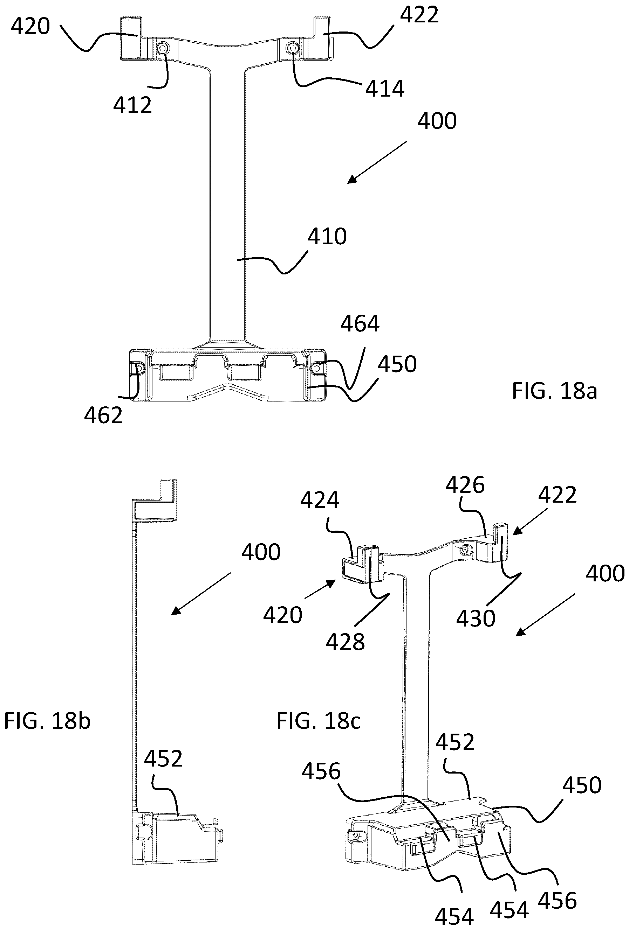

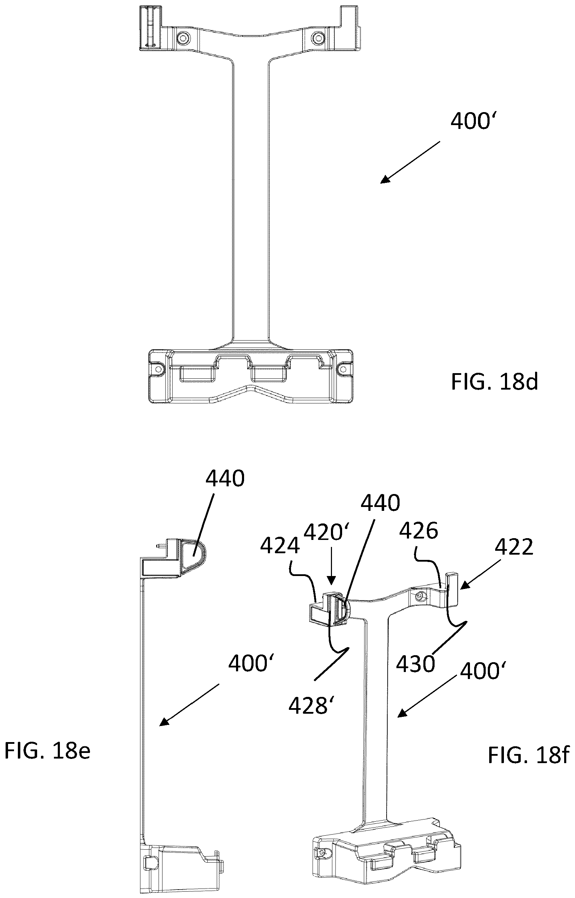

Die Figuren 18a, 18b und 18c zeigen eine in Verbindung mit den beschriebenen Notfallkoffern 100 und 200 bzw. Notfallkofferhälfte derselben verwendbare Wandhalterung 400, die Figuren 18d, 18e und 18f dieselbe Wandhalterung, nur mit Schloss.

Die Ausführungen zur Wandhalterung 400 gelten, bis auf die Ausgestaltung des linken Fingers einmal mit bzw. einmal ohne Schloss, vollständig auch für die Wandhalterung 400' mit Schloss.

Die Wandhalterung 400 umfasst eine Rückenschiene, die T-förmig ist, also eine geradlinige und im Wesentlichen gleich breite Schiene aufweist, von der zwei kleinere Schienen abzweigen, vorliegend leicht nach oben geknickt. Am Ende der beiden kleineren Schienen der T-Form ist ein verdickter Ansatz für Bohrlöcher 412, 414 vorgesehen, durch die hindurch Schraubbolzen führbar sind, um die Wandhalterung 400 an einer Wand (nicht gezeigt) zu befestigen. Die Verdickungen für die Bohrlöcher 412, 414 gehören bereits den Haltearmen 420 (links) bzw. 422 (rechts) an, die vorliegend gleich lang sind, also symmetrisch zueinander ausgestaltet sind. Der Haltearm 420 stellt, wie in Fig. 18c besonders gut zu erkennen, einen Sitz 424 für einen Abschnitt eines Notfallkoffergriffs bereit, der Haltearm 422 einen entsprechenden Sitz 426 für einen anderen Abschnitt des Griffs. Der Haltearm 420 umfasst ferner den Haltefinger 428, der Haltearm 422 den Haltefinger 430.

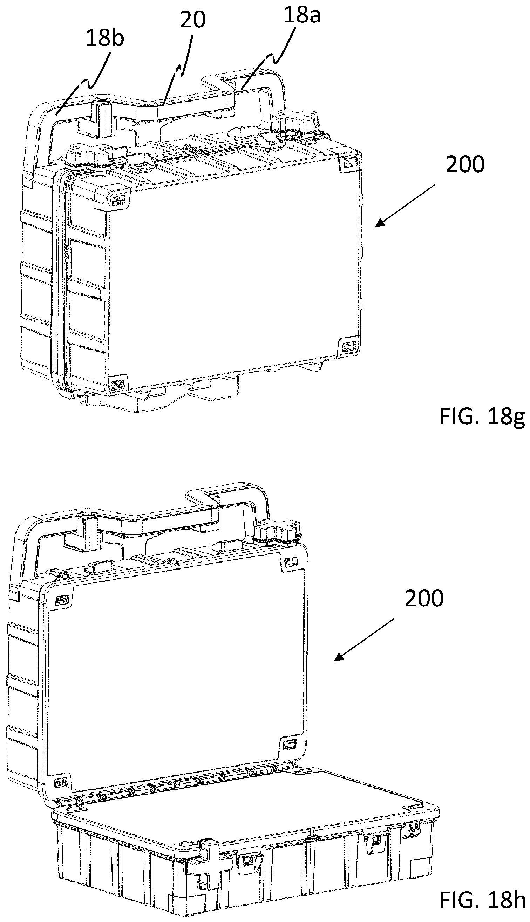

Wie anhand Fig. 18g ersichtlich, lässt sich insbesondere der Bereich 18a des Griffs 12 des Notfallkoffers 200 auf den Sitz 426 des rechten Haltearms 422 aufsetzen und der zweite Bereich 18b des Griffs auf den Sitz 424 des linken Haltearms 420 der Wandhalterung 400 aufsetzen. In Fig. 18g ist ferner auch zu erkennen, dass durch den ausreichenden Abstand zwischen den Fingern 428, 430 der Haltearme 420 bzw. 422 der dritte Bereich 20 zwischen den beiden zweiten Bereichen 18a und 18b des Griffs besonders gut zugänglich ist, was durch die Form des Griffs (der dritte Abschnitt verläuft entlang der Innenlängskante) erleichtert ist. Der gesamte Koffer lässt sich unter Ergreifen des dritten Abschnitts 20 in einem Zug herunternehmen. Im in der Zeichnung nicht dargestellten Fall, dass der Notfallkoffer 100 mit zwei Griffen verwendet wird, lässt sich naturgemäß der Notfallkoffer an den beiden dritten Bereichen 20 gemeinsam ergreifen, ähnlich wie oben in der Figur 16a zum Ersthelfer beschrieben.

Wie ferner in den Figuren 18a, 18b und 18c zu sehen, weist die Wandhalterung 400 einen Anschlag 450 für den Notfallkoffer auf. Bohrlöcher 462, 464 sind hier ebenfalls vorgesehen. Der Anschlag 450 beinhaltet zum einen einen Sitz 452, auf dem die hintere (also wandseitige) Notfallkofferhälfte aufsitzen kann. Im vorderen Bereich umfasst der Anschlag 450 Ausbuchtungen 454 und Vorsprünge 456, welche in ihren Abmessungen genau den Rippen an der Kofferschale des Notfallkoffers entsprechen, siehe Fig. 6d und die zugehörige Beschreibung der Rippen 68 passend zu den Steckeinrichtungen 66.

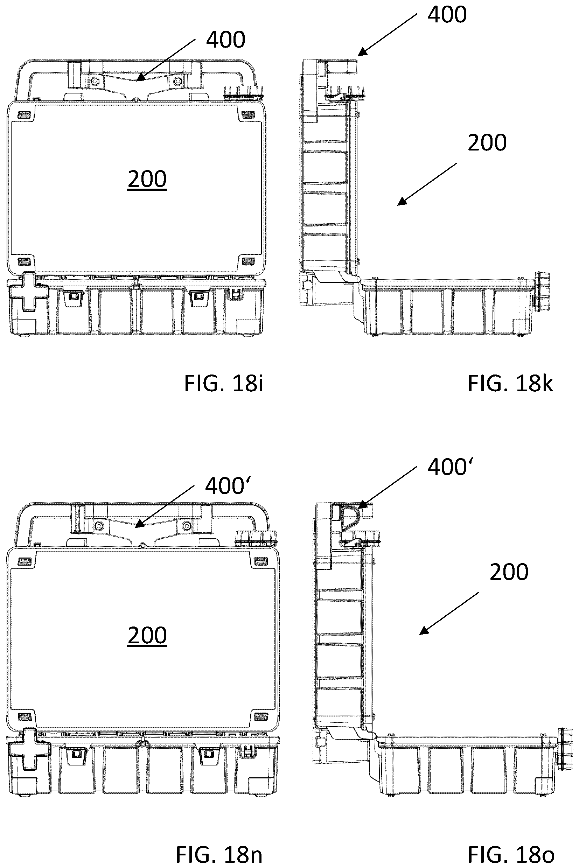

Auf diese Weise lässt sich der Notfallkoffer im aufgeklappten Zustand, wie in Fig. 18h, 18i und 18k gezeigt, besonders gut und stabil in der Wandhalterung halten bzw. vom nicht aufgeklappten Zustand gemäß Fig. 18g in den aufgeklappten Zustand gemäß Fig. 18h verbringen.

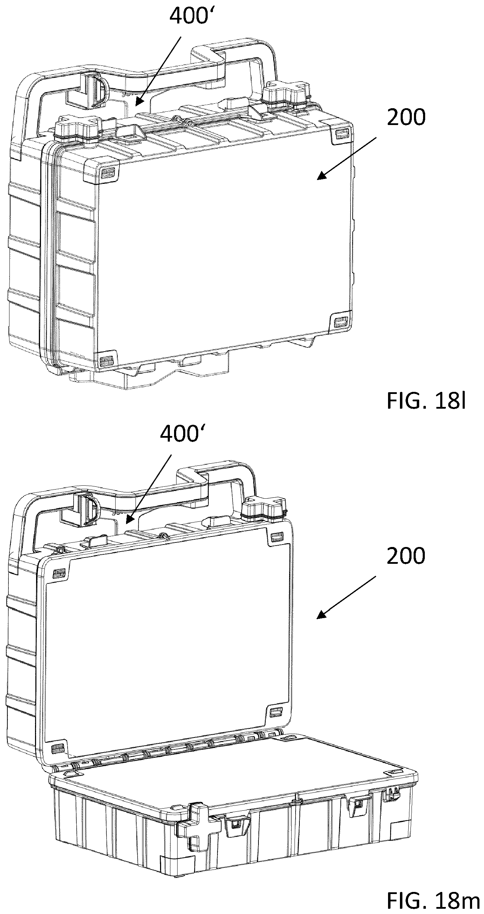

Die Wandhalterung 400', die in den Figuren 18d, 18e, 18f sowie 18l, 18m, 18n, 18o gezeigt ist, unterscheidet sich von der Wandhalterung 400 nur dadurch, dass die Verriegelung (Schloss) 440 am Finger 428' des Haltearms 420' vorgesehen ist, siehe insbesondere die Fig. 18f.

Bei dieser Ausführungsform ist durch die Verriegelung / das Schloss 440 der Notfallkoffer, wie in Fig. 18l gezeigt, mit derwandseitigen, hinteren Notfallkofferhälfte an der Wandhalterung festgehalten. Somit kann niemand diese Notfallkofferhälfte von der Wand entfernen, ohne das Schloss zu öffnen. Das sichert das Vorhandensein von entsprechendem Erste-Hilfe-Material und dergleichen, was in der Notfallkofferhälfte enthalten ist. Im aufgeklappten Zustand gemäß Fig. 18m lässt sich allerdings die vordere, heruntergeklappte Notfallkofferhälfte in der oben anhand der Figuren 12a bis 12d beschriebenen Art von der hinteren Notfallkofferhälfte trennen und später auch wieder an diese anbringen. Somit ist in dem Raum, in dem die Wandhalterung an der Wand befestigt ist, gewährleistet, dass eine Notfallkofferhälfte auf jeden Fall im Raum verbleibt, während für den Ersthelfer dringend benötigtes Material durch die andere Notfallkofferhälfte bereitgestellt sein kann. Abweichend von Fig. 18l und 18m kann auch der Notfallkoffer 100 verwendet sein, so dass die abgetrennte Notfallkofferhälfte mit einem Griff versehen sein kann.

Zwar wurde die Erfindung unter Bezug auf bestimmte Ausführungsformen beschrieben, es sollte sich aber verstehen, dass die Erfindung auf diese Beispiele nicht beschränkt ist, und dass zahlreiche Abwandlungen vom Fachmann vorgesehen und erdacht werden können, 10 nachdem er sich die Beispiele angesehen hat. Die Erfindung kann also ohne Beschränkung und beispielhaft anhand der folgenden Ausführungsformen beschrieben und so verwirklicht werden. Die folgenden Ausführungsformen können bevorzugte Ausführungsformen sein. Entsprechend bezeichnet der Begriff "Klausel" wie hierin verwendet eine solche "bevorzugte Ausführungsform"

### Klausel 1:

Notfallkoffer, mit zumindest einer Kofferschale, wobei an der Kofferschale ein Griff ausgebildet ist, der mit zwei Ansatzenden des Griffs jeweils in einer stehenden Position des Notfallkoffers an einer oberen Außenecke der Kofferschale angeordnet ist, sich in einem ersten Abschnitt vertikal nach oben erstreckt und in einem zweiten Abschnitt zu der den Ecken abgewandten Innenlängskante der Kofferschale hin erstreckt und wobei sich ein die beiden zweiten Abschnitte verbindender dritter Abschnitt des Griffs entlang der Innenlängskante erstreckt.

### Klausel 2:

Notfallkoffer nach Klausel 1, bei dem der Übergang zwischen dem ersten Abschnitt und dem zweiten Abschnitt und/oder der Übergang zwischen dem zweiten Abschnitt und dem dritten Abschnitt abgerundet ist.

### Klausel 3:

Notfallkoffer nach Klausel 1 oder 2, bei dem der zweite Abschnitt des Griffs partiell parallel zu einer Außenlängskante der Kofferschale verläuft und sich weiter dann geradlinig zu der Innenlängskante hin erstreckt.

### Klausel 4:

Notfallkoffer nach einer der Klauseln 1 bis 3, bei dem sich der dritte Abschnitt mittig entlang der Innenlängskante erstreckt.

### Klausel 5:

Notfallkoffer nach Klausel 4, bei dem jeder Griff symmetrisch ausgebildet ist.

### Klausel 6:

Notfallkoffer nach einer der Klauseln 1 bis 5 mit zwei zueinander komplementären Kofferschalen.

### Klausel 7:

Notfallkoffer nach Klausel 6, mit einem Griff an beiden Kofferschalen.

### Klausel 8:

Notfallkoffer nach Klausel 7, wobei die dritten Abschnitte der beiden Griffe einander berühren.

### Klausel 9:

Notfallkoffer nach Klausel 7 oder 8, wobei am Ende jedes Griffs eine Stapelzentriereinrichtung ausgebildet ist.

### Klausel 10:

25 Notfallkoffer nach Klausel 6, mit einem Griff an nur einer der beiden Kofferschalen, wobei an den Außenecken der Kofferschale ohne Griff jeweils eine Blindkappe vorgesehen ist.

### Klausel 11:

Notfallkoffer nach Klausel 9, wobei am Ende des Griffs eine Stapelzentriereinrichtung und wobei an den Blindkappen jeweils eine Stapelzentriereinrichtung ausgebildet ist.

### Klausel 12:

Notfallkoffer nach einer der Klauseln 9 oder 11, bei dem die beiden Stapelzentriereinrichtungen einer Kofferschale zueinander komplementär sind.

### Klausel 13:

Notfallkoffer nach einer der Klauseln 1 bis 12, bei dem jede Kofferschale Teil einer Notfallkofferhälfte ist, die mit der anderen Notfallkofferhälfte lösbar verbunden ist.

### Klausel 14:

Notfallkoffer nach einer der Klauseln 1 bis 13, wobei jede Kofferschale über einen Innendeckel verfügt.

### Klausel 15:

15 Notfallkoffer nach Klausel 14, mit Stapelzentriereinrichtungen an jedem Innendeckel.

### Klausel 16:

Notfallkoffer nach einer der Klauseln 1 bis 15, mit zumindest partiell fluoreszierend ausgebildeter und/ oder fluoreszierend bedruckter und/ oder fluoreszierend beklebter Bewandung, wobei es sich bei der Fluoreszenz insbesondere um Photolumineszenz, insbesondere mit upconversion und/oder downconversion handelt.

### Klausel 17:

Notfallkoffer nach einer der Klauseln 1 bis 16, mit zumindest partiell fluoreszierend ausgebildetem und/oder fluoreszierend bedrucktem und/ oder fluoreszierend beklebtem Griff, wobei es sich bei der Fluoreszenz insbesondere um Photolumineszenz, insbesondere mit upconversion und/oder downconversion handelt.

### Klausel 18:

Notfallkoffer mit den Merkmalen sowohl von Klausel 16 als auch von Klausel 17, bei dem der Griff heller fluoresziert als die Bewandung.

### Klausel 19:

Notfallkoffer nach einer der Klauseln 1 bis 18, befüllt mit zumindest einem aus:
- Pflaster und Verbandsmaterial;
- Einmalhandschuhen;
- Erste-Hilfe-Scheren und Pinzetten;
- Hygienemundschutz;
- 10 Hygienekleidung;
- Notduschen und Augenspülung;
- Desinfektionsmittel und Spender dazu;
- Defibrillatoren und Zubehör;
- Beatmungsmasken.

### Klausel 20:

Verwendung eines Koffers mit zumindest einer Kofferschale, wobei an der Kofferschale ein Griff ausgebildet ist, der mit zwei Ansatzenden des Griffs jeweils in einer stehenden Position des Notfallkoffers an einer oberen Außenecke der Kofferschale angeordnet ist, sich in einem ersten Abschnitt vertikal nach oben erstreckt und in einem zweiten Abschnitt zu der den Ecken abgewandten Innenlängskante der Kofferschale hin erstreckt und wobei sich ein die beiden zweiten Abschnitte verbindender dritter Abschnitt des Griffs entlang der Innenlängskante erstreckt, als Notfallkoffer.

### Klausel 21:

Verwendung des Notfallkoffers nach einer der Klauseln 1 bis 19 durch eine medizinische Behandlungsperson in einer medizinischen Notfallsituation, wobei gemäß der Verwendung ausgehend von der Situation, dass der Notfallkoffer auf einer Außenseite der Kofferschale auf dem Boden oder einer Unterlage aufliegt, an dem dritten Abschnitt von der Behandlungsperson mit der Hand ergriffen und zu der Behandlungsperson hin gezogen wird.

Die Erfindung kann auch ohne Beschränkung und beispielhaft beschrieben werden durch folgende weitere Klauseln:
Klausel 1
   Notfallkoffer, mit zwei Notfallkofferhälften, die je eine Kofferschale und einen Innendeckel aufweisen, wobei die Notfallkofferhälften lösbar verbunden sind, und wobei jeder Innendeckel zur Herstellung einer geschlossenen Notfallkofferhälfte an seiner Kofferschale arretierbar ist.
Klausel 2
   Notfallkoffer nach Klausel 1, dadurch gekennzeichnet, dass die Notfallkofferhälften ausschließlich durch an den Kofferschalen befindliche Mittel lösbar verbunden sind.
Klausel 3
   Notfallkoffer nach Klausel 1 oder 2, mit gleich gebauten Kofferschalen.
Klausel 4
   Notfallkoffer nach Klausel 3, dadurch gekennzeichnet, dass an jeder Kofferschale eine Stange und dazu achsgleich auf Umschlag eine, insbesondere im Querschnitt C-förmige, Steckvorrichtung für die Stange der anderen Kofferschale angeordnet sind.
Klausel 5
   Notfallkoffer nach Klausel 4, dadurch gekennzeichnet, dass zwischen Stange und Steckvorrichtung achsgleich verlaufende Scharniere für den jeweiligen Innendeckel der Kofferschale angeordnet sind, wobei die Scharniere der einen Kofferschale in Lücken zwischen den Scharnieren der anderen Kofferschale greifen.
Klausel 6
   Notfallkoffer nach einer der vorhergehenden Klauseln, wobei zumindest eine der Notfallkofferhälften einen Griff an der Kofferschale aufweist.
Klausel 7
   Notfallkoffer nach Klausel 6, dadurch gekennzeichnet, dass beide Notfallkofferhälften einen Griff aufweisen.
Klausel 8
   Notfallkoffer nach Klausel 6, dadurch gekennzeichnet, dass nur eine Notfallkofferhälfte einen Griff aufweist und die Kofferschale der anderen Notfallkofferhälfte zwei Blindkappen aufweist.
Klausel 9
   Notfallkoffer nach einer der Klauseln 6 bis 8, dadurch gekennzeichnet, dass jeder Griff augehend von zwei Ansatzenden jeweils an einer oberen Außenecke der jeweiligen Kofferschale sich in einem ersten Abschnitt vertikal nach oben erstreckt, in einem auf den ersten Abschnitt jeweils folgenden zweiten Abschnitt zur Innenlängskante der jeweiligen Kofferschale hin erstreckt und wobei ein dritter Abschnitt die beiden zweiten Abschnitte verbindet.
Klausel 10
   20 Notfallkoffer, mit Stapelzentriereinrichtungen.
Klausel 11
   Notfallkoffer nach einer der vorhergehenden Klauseln, dadurch gekennzeichnet, dass der Innendeckel an der zugehörigen Kofferschale verrastet ist oder verrastbar ist.
Klausel 12
   Notfallkoffer nach einer der vorhergehenden Klauseln, dadurch gekennzeichnet, dass jeder Innendeckel um 90° hochstellbar ist.
Klausel 13
   Notfallkoffer nach einer der vorhergehenden Klauseln, dadurch gekennzeichnet, dass bei geöffnetem Koffer ein Innendeckel um 180° drehbar ist, so dass er auf dem anderen Innendeckel zu liegen kommt.
Klausel 14
   Notfallkoffer nach Klausel 12 oder 13, dadurch gekennzeichnet, dass die Innendeckel miteinander verrasten, wenn beide Innendeckel um 90° hochgestellt sind bzw. ein Innendeckel um 180° gedreht ist.
Klausel 15
   Notfallkoffer nach einer der vorhergehenden Klauseln, der befüllt ist mit zumindest einem aus:
   - Pflaster und Verbandsmaterial,
   - Einmalhandschuhen,
   - Erste-Hilfe-Scheren und Pinzetten,
   - Hygienemundschutz,
   - Hygienekleidung,
   - Notduschen und Augenspülung,
   - Desinfektionsmittel und Spender dazu,
   - Defibrillatoren und Zubehör,
   - Beatmungsmasken.
Klausel 16
   Notfallkofferhälfte, mit einer Kofferschale und einem Innendeckel, wobei die Kofferschale mit einer gleich gebauten Kofferschale verbindbar ist, insbesondere werkzeuglos verbindbar ist.
Klausel 17
   Notfallkofferhälfte nach Klausel 16, die befüllt ist mit zumindest einem aus:
   - Pflaster und Verbandsmaterial,
   - Einmalhandschuhen,
   - Erste-Hilfe-Scheren und Pinzetten,
   - Hygienemundschutz,
   - Hygienekleidung,
   - Notduschen und Augenspülung,
   - Desinfektionsmittel und Spender dazu,
   - Defibrillatoren und Zubehör,
   - Beatmungsmasken.
Klausel 18
   Notfallkofferhälfte nach Klausel 16 oder 17, deren Kofferschale eine Stange und dazu achsgleich auf Umschlag eine, insbesondere im Querschnitt C-förmige, Steckvorrichtung für die Stange einer gleichgebauten Kofferschale angeordnet sind.
Klausel 19
   Notfallkofferhälfte nach einer der Klauseln 16 bis 18, bei der der Innendeckel opak ist.
Klausel 20
   Verfahren zum Verbinden zweier Notfallkofferhälften nach Klausel 18, bei dem die beiden Stangen der Notfallkofferhälften in die Steckvorrichtung der jeweils anderen Notfallkofferhälfte eingesteckt werden.
Klausel 21
   Verwendung der Notfallkofferhälfte nach einer der Klauseln 16 bis 18 einzeln als für sich tragbarer Notfallkoffer.

Die Erfindung kann des Weiteren ohne Beschränkung und beispielhaft beschrieben werden durch folgende weitere Klauseln:
Klausel 1
   Notfallkoffer mit einer ersten Kofferschale und einer zweiten Kofferschale und mit einer ersten Handhabeeinrichtung an der ersten Kofferschale, welche aus einer Ruhestellung in eine Schließstellung verbringbar ist und umgekehrt, und mit einer zweiten Handhabeeinrichtung an der zweiten Kofferschale, welche ebenfalls aus einer Ruhestellung in eine Schließstellung verbringbar ist und umgekehrt, wobei bei gegebener Ruhestellung beider Handhabeeinrichtungen der Notfallkoffer geöffnet ist und wobei in der Schließstellung der ersten Handhabeeinrichtung die erste Handhabeeinrichtung mit einem Hintergreifelement einen an der zweiten Kofferschale ausgebildeten Körper hintergreift und wobei in der Schließstellung der zweiten Handhabeeinrichtung die zweite Handhabeeinrichtung mit einem Hintergreifelement einen an der ersten Kofferschale ausgebildeten Körper hintergreift.
Klausel 2
   Notfallkoffer nach Klausel 1, bei dem die beiden Kofferschalen voneinander lösbar sind.
Klausel 3
   Notfallkoffer nach Klausel 2, mit zwei Notfallkofferhälften, die je eine Kofferschale und einen Innendeckel aufweisen, wobei die Notfallkofferhälften lösbar verbunden sind, und wobei jeder Innendeckel zur Herstellung einer geschlossenen Notfallkofferhälfte an seiner Kofferschale arretierbar ist.
Klausel 4
   Notfallkoffer nach einer der vorstehenden Klauseln 1 bis 3, bei dem jede Handhabeeinrichtung drehbar ist.
Klausel 5
   Notfallkoffer nach einer der vorstehenden Klauseln 1 bis 4, bei dem jede Handhabeeinrichtung bei nach oben offener zugehöriger Kofferschale vorne links angeordnet ist und sich im Uhrzeigersinn von der Ruhestellung in die Schließstellung drehen lässt.
Klausel 6
   Notfallkoffer nach einer der vorstehenden Klauseln 1 bis 5, bei dem jede Handhabeeinrichtung eine Handhabe umfasst, zu der ein geometrischer Mittelpunkt definierbar ist, und wobei eine Drehachse der Handhabeeinrichtung zum geometrischen Mittelpunkt der Handhabe exzentrisch liegt.
Klausel 7
   Notfallkoffer nach einer der vorstehenden Klauseln 1 bis 6, bei dem jede Handhabeeinrichtung eine kreuzförmige Handhabe aufweist, wobei vorzugsweise gleich lange Schenkel in einem Winkel von 90° zum jeweils benachbarten Schenkel stehen.
Klausel 8
   Notfallkoffer nach Klausel 7, bei dem eine Welle der Handhabeeinrichtung an einem ersten Schenkel der Kreuzform ausgebildet ist.
Klausel 9
   Notfallkoffer nach Klausel 8, bei dem das Hintergreifelement an dem dem ersten Schenkel gegenüberliegenden Schenkel der Kreuzform ausgebildet ist.
Klausel 10
   Notfallkoffer nach einer der vorstehenden Klauseln 1 bis 9, bei dem der von einer Handhabeeinrichtung hintergriffene oder hintergreifbare Körper als Nase ausgebildet ist und vorzugsweise einen Rastmechanismus aufweist.
Klausel 11
   Notfallkoffer nach einer der vorstehenden Klauseln 1 bis 10, bei dem jede Handhabeeinrichtung zur Herstellung der Schließstellung ausgehend von der Ruhestellung in eine erste Drehrichtung, vorzugsweise um 90°, zu verdrehen ist, und bei dem jede Handhabeeinrichtung ausgehend von der Ruhestellung in eine zweite Drehrichtung entgegengesetzt zur ersten Drehrichtung zur Herstellung einer Parkstellung verdrehbar ist, vorzugsweise um 90°.
Klausel 12
   Notfallkoffer nach einer der vorstehenden Klauseln 1 bis 11, bei dem zumindest an einer Kofferschale ein Griff ausgebildet ist, der mit zwei Ansatzenden des Griffs jeweils in einer stehenden Position des Notfallkoffers an einer oberen Außenecke der Kofferschale angeordnet ist, sich in einem ersten Abschnitt vertikal nach oben erstreckt und in einem zweiten Abschnitt zu der den Ecken abgewandten Innenlängskante der Kofferschale hin erstreckt und wobei sich ein die beiden zweiten Abschnitte verbindender dritter Abschnitt des Griffs entlang der Innenlängskante erstreckt.
Klausel 13
   Notfallkoffer nach einer der vorstehenden Klauseln 1 bis 12, der befüllt ist mit zumindest einem aus:
   - Pflaster und Verbandsmaterial,
   - Einmalhandschuhen,
   - Erste-Hilfe-Scheren und Pinzetten,
   - Hygienemundschutz,
   - Hygienekleidung,
   - Notduschen und Augenspülung,
   - Desinfektionsmittel und Spender dazu,
   - Defibrillatoren und Zubehör,
   - Beatmungsmasken.
Klausel 14
   Notfallkofferhälfte mit einer Kofferschale, mit einer Handhabeeinrichtung und mit einem hintergreifbaren Körper, wobei die Notfallkofferhälfte mit einer gleichartigen Kofferschale mit Handhabeeinrichtung und hintergreifbarem Körper verbindbar ist, um einen Notfallkoffer bereitzustellen, bei dem durch jede Handhabeeinrichtung der einen Kofferschale der hintergreifbare Körper an der anderen Kofferschale hintergreifbar ist.
Klausel 15
   Notfallkofferhälfte nach Klausel 14, die befüllt ist mit zumindest einem aus:
   - Pflaster und Verbandsmaterial,
   - Einmalhandschuhen,
   - Erste-Hilfe-Scheren und Pinzetten,
   - Hygienemundschutz,
   - Hygienekleidung,
   - Notduschen und Augenspülung,
   - Desinfektionsmittel und Spender dazu,
   - Defibrillatoren und Zubehör,
   - Beatmungsmasken.

Besonders vorteilhaft ist ein Gegenstand, der wenigstens einen der nachfolgenden Aspekte aufweist:
1. Notfallkoffer (100. 200) mit zwei Notfallkofferhälften, die je eine Kofferschale (10, 10') aufweisen, wobei in zumindest einer Kofferschale (10, 10') Trennelemente (300a, 300b, 300c, 300d) zur Bildung von Fächern bereitgestellt sind, wobei unmittelbar als Teil von zumindest zwei Innenwänden dieser Kofferschale je eine Folge von Steckeinrichtungen (66) bereitgestellt ist und wobei die Trennelemente (300a, 300b, 300c, 300d) zumindest in einem Teil der Steckeinrichtungen eingesteckt sind.
2. Notfallkoffer nach Aspekt 1 mit rechteckigen Kofferschalen (10, 10') und geradlinig verlaufenden Trennelementen (300a, 300b, 300c, 300d), die je an einander gegenüberliegenden Steckeinrichtungen (66) einer Kofferschale (10, 10') eingesteckt sind.
3. Notfallkoffer nach Aspekt 1 oder 2, bei dem zumindest einer und vorzugsweise jeder Steckeinrichtung (66) an der Innenwand der Kofferschale (10, 10') eine Verstärkungsrippe (68) an der Außenwand örtlich entspricht.
4. Notfallkoffer nach einem der Aspekte 1 bis 3, bei dem jede Kofferschale (10, 10') und/ oder jedes Trennelement in einem Stück aus Hartplastik hergestellt ist.
5. Notfallkoffer nach einem der Aspekte 1 bis 4, bei dem die Trennelemente (300a, 300b, 300c, 300d) einen streifenförmigen Grundkörper (310a, 310b, 310c, 310d) aufweisen und bei dem an zumindest einer Seite des streifenförmigen Grundkörpers (310a, 310b, 310c, 310d) eine Folge von Steckeinrichtungen (320-1, 320-2) für andere Trennelemente bereitgestellt sind.
6. Notfallkoffer nach Aspekt 5, bei dem die Steckeinrichtungen (320-1, 320-2) der Trennelemente (300a, 300b, 300c, 300d) jeweils mehrere Nasen (322) aufweisen, die alternierend rechts und links untereinander angeordnet sind.
7. Notfallkoffer nach einem der Aspekte 1 bis 6, bei dem die Trennelemente (300a, 300b, 300c, 300d) an zumindest einem Längsende eine Schiebeschiene (330) aufweisen, die in eine Steckeinrichtung einbringbar ist.
8. Notfallkoffer nach einem der Aspekte 1 bis 7, der befüllt ist mit zumindest einem aus:
   - Pflaster und Verbandsmaterial;
   - Einmalhandschuhen;
   - Erste-Hilfe-Scheren und Pinzetten;
   - Hygienemundschutz;
   - Hygienekleidung;
   - Notduschen und Augenspülung;
   - Desinfektionsmittel und Spender dazu;
   - Defibrillatoren und Zubehör;
   - Beatmungsmasken.
9. Notfallkoffer (100, 200) nach einem der Aspekte 1 bis 8, bei dem in jeder Kofferschale (10, 10') Trennelemente (300a, 300b, 300c, 300d) zur Bildung von Fächern bereitgestellt sind.
10. Notfallkofferhälfte mit einer Kofferschale (10, 10') und einer unmittelbar als Teil von zumindest zwei Innenwänden der Kofferschale (10, 10') jeweils angeordneten Folge von Steckeinrichtungen (66), wobei in zumindest einem Teil der Steckeinrichtungen (66) Trennelemente (300a, 300b, 300c, 300d) zur Bildung von Fächern eingesteckt sind.
11. Notfallkofferhälfte nach Aspekt 10 mit einem Innendeckel (26), wobei der Innendeckel (26) zur Herstellung einer geschlossenen Notfallkofferhälfte an der Kofferschale (10, 10') arretierbar ist.
12. Notfallkofferhälfte nach Aspekt 10 oder 11, die befüllt ist mit zumindest einem aus:
   - Pflaster und Verbandsmaterial;
   - Einmalhandschuhen;
   - Erste-Hilfe-Scheren und Pinzetten;
   - Hygienemundschutz;
   - Hygienekleidung;
   - Notduschen und Augenspülung;
   - Desinfektionsmittel und Spender dazu;
   - Defibrillatoren und Zubehör;
   - Beatmungsmasken.
13. System von Notfallkoffern (100a, 100b, 100c) mit den Merkmalen nach einem der Aspekte 1 bis 9 in verschiedenen Größen und/oder von Notfallkofferhälften mit den Merkmalen nach einem der Aspekte 10 bis 12 in verschiedenen Größen, wobei die Abmessungen der Kofferschalen (10, 10') mit den Steckeinrichtungen (66) untereinander und mit den Abmessungen der Trennelemente (300a, 300b, 300c, 300d) derart aufeinander abgestimmt sind, dass vorbestimmte Trennelemente (300a, 300b, 300c, 300d) in mehreren Notfallkoffern (100a, 100b, 100c) und/ oder Notfallkofferhälften mit unterschiedlich großen Kofferschalen (10, 10') nutzbar sind.
14. System nach Aspekt 13, bei dem die vorbestimmten Trennelemente (300a, 300b, 300c, 300d) eine Gruppe als Bausatz bilden, der bei allen Notfallkoffern (100a, 100b, 100c) oder Notfallkofferhälften des Systems nutzbar ist.
15. Trennelement (300a, 300b, 300c, 300d) für eine Kofferschale (10, 10') eines Notfallkoffers (100, 200) und mit einem streifenförmigen Grundkörper (310a, 310b, 310c, 310d), an dem Steckeinrichtungen (320-1, 320-2) ausgebildet sind, wobei jede Steckeinrichtung (320-1, 320-2) Nasen (322) aufweist, welche alternierend rechts und links untereinander angeordnet sind.
16. Trennelement nach Aspekt 15, bei dem auf jeder Seite des streifenförmigen Grundkörpers (310a, 310b, 310c, 310d) Steckeinrichtungen (320-1, 320-2) bereitgestellt sind, wobei jeder Nase (322) der einen Seite eine Aussparung (324) auf dieser einen Seite gegenüberliegt, welcher eine Nase an der anderen Seite entspricht, und wobei der Nase (322) der einen Seite die entsprechende Aussparung auf der anderen Seite entspricht, welche der Nase auf der anderen Seite gegenüberliegt.

### Bezugszeichenliste:

- 1: Ersthelfer
- 1H: Hand des Ersthelfers 1
- 2: Patient
- 10: Kofferschale
- 10': Kofferschale
- 12: Griff
- 14a: obere Außenecke
- 14b: obere Außenecke
- 16a: erster Abschnitt
- 16b: erster Abschnitt
- 18a: zweiter Abschnitt
- 18b: zweiter Abschnitt
- 20: dritter Abschnitt
- 22: Innenlängskante
- 24: Außenlängskante
- 26: Innendeckel
- 28: Pfeil
- 30a: Stapelzentriereinrichtung
- 30b: Stapelzentriereinrichtung
- 32a: Stapelzentriereinrichtung
- 32b: Stapelzentriereinrichtung
- 34a: Stapelzentriereinrichtung
- 34b: Stapelzentriereinrichtung
- 36a: Stapelzentriereinrichtung
- 36b: Stapelzentriereinrichtung
- 38a: Stapelzentriereinrichtung
- 38b: Stapelzentriereinrichtung
- 40a: Stapelzentriereinrichtung
- 40b: Stapelzentriereinrichtung
- 42a: Blindkappe
- 42b: Blindkappe
- 44a: Stapelzentriereinrichtung
- 44b: Stapelzentriereinrichtung
- 50: Stange
- 52: Steckvorrichtung
- 54a: Einrichtung
- 54b: Einrichtung
- 54c: Einrichtung
- 56a: Lücke
- 56b: Lücke
- 56c: Lücke
- 57a: Drehbewegung angebender Pfeil
- 57b: Drehbewegung angebender Pfeil
- 58a: Klemmlasche
- 58b: Klemmlasche
- 59a: Rastnase
- 59b: Rastnase
- 60: ebene Fläche
- 62: Vorsprung
- 64: Ausnehmung
- 66: Steckeinrichtung
- 68: Verstärkungsrippe
- 70: oberes Rahmenteil des Griffs 12
- 72: unteres Rahmenteil des Griffs 12
- 74: Einsatzteil des Griffs 12
- 76: Kavität
- 78: kreisförmige Aussparungen
- 80: leistenförmiger Grundkörper des Einsatzteils 74
- 82: Noppen
- 84: Stecklasche
- 86: Elektronikmodul
- 90a: Handhabeeinrichtung
- 90b: Handhabeeinrichtung
- 92: Handhabe
- 93a: Schenkel der Handhabe
- 93b: Schenkel der Handhabe
- 94: Hohlwelle
- 95: Flansch
- 96: Hintergreifnase
- 98: Rastnase
- 99a: Drehrichtung der Handhabeeinrichtung 90a beim Schließen
- 99b: Drehrichtung der Handhabeeinrichtung 90b beim Schließen
- 100: Notfallkoffer
- 100a, 100b, 100c: Notfallkoffer verschiedener Größe
- 110: Kofferschale
- 181a: Teilabschnitt
- 181b: Teilabschnitt
- 182a: Teilabschnitt
- 182b: Teilabschnitt
- 200: Notfallkoffer
- 300a: Trennelement
- 300b: Trennelement
- 300c: Trennelement
- 300d: Trennelement
- 310a: streifenförmiger Grundkörper
- 310b: streifenförmiger Grundkörper
- 310c: streifenförmiger Grundkörper
- 310d: streifenförmiger Grundkörper
- 320-1: Steckeinrichtung
- 320-2: Steckeinrichtung
- 322: Nasen
- 324: Aussparungen
- 330: Schiebeschiene
- 400: Wandhalterung ohne Schloss
- 400': Wandhalterung mit Schloss
- 410: T-förmige Rückenschiene
- 412: Ansatz für Bohrlöcher
- 414: Ansatz für Bohrlöcher
- 420: Haltearm
- 420': Haltearm
- 422: Haltearm
- 424: Sitz am Haltearm 420
- 426: Sitz am Haltearm 422
- 428: Finger am Haltearm 420
- 428': Finger am Haltearm 420'
- 430: Finger am Haltearm 422
- 440: Schloss
- 450: Anschlag
- 452: Sitz des Anschlags 450
- 454: Ausbuchtung
- 456: Vorsprung
- 462: Bohrloch
- 464: Bohrloch
- a1: Anstellwinkel
- a2: Anstellwinkel
- β1: Winkel
- β2: Winkel
- d3: Länge des Abschnitts 20
- dges: Gesamtlänge
- M: geometrischer Mittelpunkt der Handhabe 92

## Patentansprüche

1. System von
a. Notfallkoffern (100a, 100b, 100c) in verschiedenen Größen mit jeweils zwei Notfallkofferhälften, die je eine Kofferschale (10, 10') aufweisen, wobei in zumindest einer Kofferschale (10, 10') Trennelemente (300a, 300b, 300c, 300d) zur Bildung von Fächern bereitgestellt sind, wobei unmittelbar als Teil von zumindest zwei Innenwänden dieser Kofferschale je eine Folge von Steckeinrichtungen (66) bereitgestellt ist und wobei die Trennelemente (300a, 300b, 300c, 300d) zumindest in einem Teil der Steckeinrichtungen eingesteckt sind, und/oder von
b. Notfallkofferhälften in verschiedenen Größen mit jeweils einer Kofferschale (10, 10') und einer unmittelbar als Teil von zumindest zwei Innenwänden der Kofferschale (10, 10') jeweils angeordneten Folge von Steckeinrichtungen (66), wobei in zumindest einem Teil der Steckeinrichtungen (66) Trennelemente (300a, 300b, 300c, 300d) zur Bildung von Fächern eingesteckt sind, **dadurch gekennzeichnet, dass**
die Abmessungen der Kofferschalen (10, 10') mit den Steckeinrichtungen (66) untereinander und mit den Abmessungen der Trennelemente (300a, 300b, 300c, 300d) derart aufeinander abgestimmt sind, dass vorbestimmte Trennelemente (300a, 300b, 300c, 300d) in mehreren Notfallkoffern (100a, 100b, 100c) und/ oder Notfallkofferhälften mit unterschiedlich großen Kofferschalen (10, 10') nutzbar sind.

2. System nach Anspruch 1 mit rechteckigen Kofferschalen (10, 10') und geradlinig verlaufenden Trennelementen (300a, 300b, 300c, 300d), die je an einander gegenüberliegenden Steckeinrichtungen (66) einer Kofferschale (10, 10') eingesteckt sind.

3. System nach Anspruch 1 oder 2, bei dem zumindest einer und vorzugsweise jeder Steckeinrichtung (66) an der Innenwand der Kofferschale (10, 10') eine Verstärkungsrippe (68) an der Außenwand örtlich entspricht.

4. System nach einem der Ansprüche 1 bis 3, bei dem jede Kofferschale (10, 10') und/ oder jedes Trennelement in einem Stück aus Hartplastik hergestellt ist.

5. System nach einem der Ansprüche 1 bis 4, bei dem die Trennelemente (300a, 300b, 300c, 300d) einen streifenförmigen Grundkörper (310a, 310b, 310c, 310d) aufweisen und bei dem an zumindest einer Seite des streifenförmigen Grundkörpers (310a, 310b, 310c, 310d) eine Folge von Steckeinrichtungen (320-1, 320-2) für andere Trennelemente bereitgestellt sind.

6. System nach Anspruch 5, bei dem die Steckeinrichtungen (320-1, 320-2) der Trennelemente (300a, 300b, 300c, 300d) jeweils mehrere Nasen (322) aufweisen, die alternierend rechts und links untereinander angeordnet sind.

7. System nach einem der Ansprüche 1 bis 6, bei dem die Trennelemente (300a, 300b, 300c, 300d) an zumindest einem Längsende eine Schiebeschiene (330) aufweisen, die in eine Steckeinrichtung einbringbar ist.

8. System nach einem der Ansprüche 1 bis 7, die Notfallkoffer (100a, 100b, 100c) befüllt sind mit zumindest einem aus:
- Pflaster und Verbandsmaterial;
- Einmalhandschuhen;
- Erste-Hilfe-Scheren und Pinzetten;
- Hygienemundschutz;
- Hygienekleidung;
- Notduschen und Augenspülung;
- Desinfektionsmittel und Spender dazu;
- Defibrillatoren und Zubehör;
- Beatmungsmasken.

9. System (100, 200) nach einem der Ansprüche 1 bis 8, bei dem in jeder Kofferschale (10, 10') Trennelemente (300a, 300b, 300c, 300d) zur Bildung von Fächern bereitgestellt sind.

10. System nach Anspruch 1, **dadurch gekennzeichnet, dass** die Notfallkofferhälften jeweils einen Innendeckel (26) aufweisen, der zur Herstellung einer geschlossenen Notfallkofferhälfte an der Kofferschale (10, 10') arretierbar ist.

11. System nach Anspruch 10, **dadurch gekennzeichnet, dass** die Notfallkofferhälften jeweils befüllt sind mit zumindest einem aus:
- Pflaster und Verbandsmaterial;
- Einmalhandschuhen;
- Erste-Hilfe-Scheren und Pinzetten;
- Hygienemundschutz;
- Hygienekleidung;
- Notduschen und Augenspülung;
- Desinfektionsmittel und Spender dazu;
- Defibrillatoren und Zubehör;
- Beatmungsmasken.

12. System nach einem der Ansprüche 1 bis 11, bei dem die vorbestimmten Trennelemente (300a, 300b, 300c, 300d) eine Gruppe als Bausatz bilden, der bei allen Notfallkoffern (100a, 100b, 100c) oder Notfallkofferhälften des Systems nutzbar ist.

13. Trennelement (300a, 300b, 300c, 300d) für eine Kofferschale (10, 10') eines Notfallkoffers (100, 200) eines Systems nach einem der Ansprüche 1 bis 12, **dadurch gekennzeichnet, dass** das Trennelement (300a, 300b, 300c, 300d) einen streifenförmigen Grundkörper (310a, 310b, 310c, 310d) aufweist, an dem Steckeinrichtungen (320-1, 320-2) ausgebildet sind, wobei jede Steckeinrichtung (320-1, 320-2) Nasen (322) aufweist, welche alternierend rechts und links untereinander angeordnet sind.

14. Trennelement nach Anspruch 13, bei dem auf jeder Seite des streifenförmigen Grundkörpers (310a, 310b, 310c, 310d) Steckeinrichtungen (320-1, 320-2) bereitgestellt sind, wobei jeder Nase (322) der einen Seite eine Aussparung (324) auf dieser einen Seite gegenüberliegt, welcher eine Nase an der anderen Seite entspricht, und wobei der Nase (322) der einen Seite die entsprechende Aussparung auf der anderen Seite entspricht, welche der Nase auf der anderen Seite gegenüberliegt.
